# EUROPEAN PATENT SPECIFICATION

(11) **EP 3 298 167 B1**
(45) Date of publication and mention of the grant of the patent: **20.01.2021**
(21) Application number: 16796852.8
(22) Date of filing: 19.05.2016
(51) Int. Cl.: C12Q 1/68

(54) **GENE EXPRESSION PROFILES AND USES THEREOF IN BREAST CANCER**
GENEXPRESSIONSPROFILE UND VERWENDUNGEN DAVON BEI BRUSTKREBS
PROFILS D'EXPRESSION GÉNIQUE ET UTILISATIONS DE CEUX-CI DANS LE CANCER DU SEIN

(30) Priority: 19.05.2015 TW 104115832
(43) Date of publication of application: 28.03.2018
(73) Proprietor: Amwise Diagnostics Pte. Ltd., Singapore 169074 (SG)
(72) Inventor: CHENG, Hung-Chun, Singapore 169074 (SG)
(74) Representative: Mewburn Ellis LLP
(86) International application number: PCT/SG2016/050238
(87) International publication number: WO 2016/186582

(56) References cited:
- WO-A1-2014/006796
- WO-A2-2006/110264
- WO-A2-2008/094678
- WO-A2-2012/079059
- WO-A2-2013/132354
- CHENG, S. H. ET AL.: 'Genomic prediction of locoregional recurrence after mastectomy in breast cancer' JOURNAL OF CLINICAL ONCOLOGY vol. 24, no. 28, 2006, pages 4594 - 4602, XP009076923
- CHENG, S. H. ET AL.: 'An Eighteen- Gene Classifier Predicts Locoregional Recurrence in Post-Mastectomy Breast Cancer Patients' EBIOMEDICINE vol. 5, 2016, pages 74 - 81, XP055374462
- MAMOUNAS, E.P. ET AL.: 'Association between the 21- gene recurrence score assay and risk of locoregional recurrence in node-negative, estrogen receptor-positive breast cancer: results from NSABP B-14 and NSABP B-20' JOURNAL OF CLINICAL ONCOLOGY vol. 28, no. 10, 2010, pages 1677 - 1683, XP055374465
- VAN 'T VEER, L.J. ET AL.: 'Gene expression profiling predicts clinical outcome of breast cancer' NATURE vol. 415, 2002, pages 530 - 536, XP008138701

## Description

### FIELD OF THE INVENTION

The present invention relates generally to gene expression profiles and uses thereof in breast cancer and in particular, but not exclusively, to a method for predicting local and/or regional (locoregional) recurrence (LRR) and/or distant metastasis risks based on gene expression profiles in a subject with breast cancer after mastectomy and/or breast conserving surgery (BCS). Particularly, but not exclusively, the present invention provides a method relating to measuring expression levels of at least one gene related to breast cancer and deriving a score based on a pre-determined model for predicting locoregional recurrence and/or distant metastasis risks in a subject with breast cancer after mastectomy and/or breast conserving surgery, which facilitates the determination of the type of treatment required after mastectomy and /or breast conserving surgery.

### BACKGROUND OF THE INVENTION

The following discussion of the background to the invention is intended to facilitate an understanding of the present invention.

A conventional method for calculating the locoregional recurrence (LRR) risk of breast cancer to determine whether radiotherapy is required is based on clinical risk factors that are found to be closely related to locoregional recurrence such as tumor size, involvement of axillary lymph nodes (axillary LN), estrogen receptor status (ER status), age of diagnosis, lymphovascular invasion (LV invasion), and the like. [1-2]. Although some studies have reported that there are individual genes related to locoregional recurrence, there are no reliable biological markers to date that may predict locoregional recurrence [3-4].

In clinical practice, the strategy for reducing locoregional recurrence of breast cancer is to give post-operative radiotherapy, whereas the strategy for diminishing distant metastasis is to give systemic adjuvant chemotherapy and/or hormonal therapy. It is generally accepted that patients with involvement of four or more axillary LNs should be given post-mastectomy radiotherapy (PMRT) [5].

For patients with 1-3 positive nodes, the National Comprehensive Cancer Network (NCCN) guidelines recommend counseling clinicians to "strongly consider" giving PMRT based on the results of three large randomized control trials which have proven the benefits of PMRT in such patients [6-8]. However, it is also reported that the locoregional recurrence rates at 10 years in node-negative patients is lower than 5%, for patients with 1-3 positive nodes about 20% and for patients with 4 or more positive nodes about 32% [9]. Therefore, based on clinical parameters, there are about 70-80% of node-positive patients who would potentially be free from locoregional recurrence after mastectomy and would not require PMRT, whereas those at risk would potentially benefit from it.

Recent progress in genomic analysis as a potential tool for evaluating tumor biology opens a new possibility of improving risk stratification that would lead to more personalized prognostication for breast cancer patients with 1-3 positive nodes [10]. For instance, Cheng, S.H., et al. attempted to develop different gene sets using unsupervised clustering and Bayesian tree model. Furthermore, the gene sets were used to distinguish N0 and N1 patients into a high-risk group and a low-risk group of locoregional recurrence to calculate their potential 3-year locoregional recurrence rates [11].

On the other hand, Solin, L.J., et al. report a 12-gene expression assay capable of predicting locoregional recurrence for ductal carcinoma in situ (DCIS) patients after wide local excision, a non-invasive carcinoma [12].

U.S. Patent No. 8741605B2 discloses an evaluation method which uses gene expression markers of breast cancer patients. The method uses a gene sequence as a precursor of SEQ ID No. 162 to make a quantitative comparison between the RNA translation level of CD68 in a breast cancer patient and the RNA translation level of CD68 in a breast cancer sample for evaluating or predicting whether a breast cancer patient may survive a long time without risk of distal recurrence. This evaluation method uses a CD68 genomic prognostic kit and refers to the statistical data of genes in a patient for evaluating the patient's survival rate. However, the genes used are too diverse and the parameters are also diverse as the statistical values obtained are greater than 1.01.

WO Publication No. 2014/085653A1 discloses the use of multiple genes to evaluate whether to intervene and treat a patient with adjuvant chemotherapy. The evaluation method disclosed uses a 14-gene set and it helps the medical personnel to determine whether a breast cancer patient needs further adjuvant chemotherapy, or to evaluate whether a breast cancer patient is at risk of distant metastases. However, it targets only a particular group of patients, which consists of postmenopausal women having breast cancer.

The identification of individuals after mastectomy and/or breast conversing surgery at risk of locoregional recurrence and/or distant metastasis is crucially important as accurate prediction of such risks will immediately impact adjuvant treatment decisions. Improving risk stratification enables a more precise prediction of risks in an individual which helps to reduce or prevent excessive treatment or overtreatment by identifying individuals with low risk of recurrence and/or distant metastasis for whom adjuvant therapies such as PMRT, regional node irradiation (RNI), chemotherapy and hormonal therapy can be avoided.

The key to prevent early breast cancer overtreatment and tailor personalized treatment is to utilize molecular diagnostics to determine whether the cancer will be indolent or aggressive [13, 14]. Although there exist certain gene panels to predict the possibility of locoregional recurrence or distant metastasis for breast cancer patients as discussed above, these are limited to particular groups of patients and for particular types of breast cancer. Evaluation of the likelihood of locoregional recurrence and/or distant metastasis in breast cancer patients across different subtypes, stages or treatment modalities is not available to date. Furthermore, the risk stratification of non-luminal breast cancer by current genomic panel is limited to date [15].

Therefore, there is a need for a method for predicting locoregional recurrence and/or distant metastasis risks in breast cancer patients after mastectomy and/or breast conserving surgery that overcomes at least in part some of the aforementioned disadvantages

### SUMMARY OF THE INVENTION

Throughout this document, unless otherwise indicated to the contrary, the terms "comprising", "consisting of', and the like, are to be construed as non-exhaustive, or in other words, as meaning "including, but not limited to".

In accordance with a first aspect of the present disclosure there is provided a method for predicting the likelihood of locoregional recurrence (LRR) and/or distant metastasis in a subject with breast cancer following mastectomy and/or breast conserving surgery, comprising:
i. measuring the expression level of at least one gene in a sample isolated from the subject; and
ii. deriving a score based on the measured expression level of the at least one gene;
   wherein the at least one gene is selected from a group consisting of: TRPV6, DDX39, BUB1B, CCR1, STIL, BLM, C16ORF7, TPX2, PTI1, TCF3, CCNB1, DTX2, PIM1, ENSA, RCHY1, NFATC2IP, OBSL1, MMP15, and a fragment, a homologue, a variant or a derivative thereof; and
   wherein the derived score provides an indication of the likelihood of LRR and/or the likelihood of distant metastasis in the subject.

The step of deriving a score based on the measured expression level of the at least one gene is performed using a predictive classification model.

Preferably, the predictive classification model comprises at least one scoring algorithm.

The method comprises a step of classifying the subject into a low risk group of LRR and/or distant metastasis when the derived score is less than a first pre-determined reference;

into a high risk group of LRR and/or distant metastasis when the derived score is equal to or more than the first pre-determined reference;

into a low risk group of distant metastasis and/or LRR when the derived score is less than a second pre-determined reference;

into a high risk group of distant metastasis and/or LRR when the derived score is equal to or more than a third pre-determined reference;

into an intermediate risk group of distant metastasis and/or LRR when the derived score is between the second pre-determined reference (inclusive) and the third pre-determined reference.

Preferably, the at least one scoring algorithm is selected from a group consisting of:
i. Score = TRPV6 + DDX39 + BUB1B + CCR1 + STIL + BLM + C16ORF7 + TPX2 + PTI1 + TCF3 + CCNB1 + DTX2 + PIM1 + ENSA + RCHY1 + NFATC2IP + OBSL1 + MMP15; wherein each gene counts as one point when the hazard ratio is <1, and the total score = 18;
ii. Score = TRPV6 + DDX39 + BUB1B + CCR1 + STIL + BLM + 2×C16ORF7 + TPX2 + PTI1 + TCF3 + CCNB1 + DTX2 + PIM1 + ENSA + RCHY1 + 2×NFATC2IP + OBSL1 + 2×MMP15; wherein when the hazard ratio is <1, each gene counts as one point if the genes are examined by univariate analysis, and each gene counts as two points if the genes are examined by multivariate analysis, and so the total score = 21;
iii. Score = 3×TRPV6 + 5×DDX39 + 18×BUB1B + 4×CCR1 + 4×STIL + 7×BLM + 7×C16ORF7 + 4×PMI1 + 9×TPX2 + 8×PTI1 + 3×TCF3 + 7×CCNB1 + 2×DTX2 + 2×ENSA + 5×RCHY1 + 6×NFATC2IP + 2×OBSL1 + 6×MMP15; wherein the genes are examined by univariate analysis and the score of each gene is re-scaled according to its weighting, and so the total score = 102;
iv. Score = 2×TRPV6 + 2×DDX39 + 2×BUB1B + CCR1 + STIL + 2×BLM + 5×C16ORF7 + 3×PIM1 + 3×TPX2 + 5×PTI1 + TCF3 + CCNB1 + DTX2 + 2×ENSA + 3×RCHY1 + 4×NFATC2IP + OBSL1 + MMP15; wherein the genes are examined by multivariate analysis and the odds ratio of each gene is re-scaled to a score between 1 and 5, each gene counts as 1 point when the odds ratio is
   i. <1, and so the total score = 40; and
   ii. Score = 4×TRPV6 + 3×DDX39 + 8×BUB1B + CCR1 + STIL + 3×BLM + 11×C160RF7 + 4×PIM1 + TPX2 + 2×PTI1 + 2×TCF3 + CCNB1 + DTX2 + 2×ENSA+ 5×RCHY1 + 4×NFATC2IP + OBSL1 + 2×MMP15; wherein the genes are examined by multivariate analysis and the odds ratio of each gene is re-scaled to a score between 1 and 11, each gene counts as 1 point when the odds ratio is <1, and so the total score = 56.

Preferably, the step of measuring the expression level of the at least one gene comprises hybridizing the at least one gene with at least one gene probe and measuring the expression level of the at least one gene.

Preferably, the at least one gene probe comprises at least one gene selected from a group consisting of: TRPV6, DDX39, BUB1B, CCR1, STIL, BLM, C16ORF7, TPX2, PTI1, TCF3, CCNB1, DTX2, PIM1, ENSA, RCHY1, NFATC2IP, OBSL1, MMP15, and a fragment, a homologue, a variant or a derivative thereof.

Preferably, the at least one gene probe is fixed on a microarray chip.

Preferably, the measurement of gene expression level is performed by a microarray or quantitative reverse transcriptase polymerase chain reaction (quantitative RT-PCR).

The first pre-determined reference is a score of 31.

The second pre-determined reference is a score of 21.

The third pre-determined reference is a score of 44.

Preferably, the subject has one of the following conditions: zero nodes, one to three positive nodes, and more than three positive nodes.

In accordance with a second aspect of the present disclosure, there is provided a kit for predicting the likelihood of locoregional recurrence (LRR) and/or distant metastasis in a subject with breast cancer following mastectomy and/or breast conversing surgery, comprising:
i. at least one reagent capable of specifically binding to at least one gene in a sample isolated from the subject to quantify the expression level of the at least one gene; and
ii. a predictive classification model comprising at least one scoring algorithm for deriving a score based on the expression level of the at least one gene,
wherein the at least one gene is selected from a group consisting of: TRPV6, DDX39, BUB1B, CCR1, STIL, BLM, C16ORF7, TPX2, PTI1, TCF3, CCNB1, DTX2, PIM1, ENSA, RCHY1, NFATC2IP, OBSL1, MMP15, and a fragment, a homologue, a variant or a derivative thereof; and
wherein the derived score provides an indication of the likelihood of LRR and/or the likelihood of distant metastasis in the subject.

In accordance with a third aspect of the present disclosure, there is provided amicroarray for predicting the likelihood of locoregional recurrence (LRR) and/or distant metastasis in a subject with breast cancer following mastectomy and/or breast conserving surgery, comprising at least one gene probe for measuring the expression level of at least one gene selected from a group consisting of: TRPV6, DDX39, BUB1B, CCR1, STIL, BLM, C16ORF7, TPX2, PTI1, TCF3, CCNB1, DTX2, PIM1, ENSA, RCHY1, NFATC2IP, OBSL1, MMP15, and a fragment, a homologue, a variant or a derivative thereof.

Preferably, the predicted likelihood of LRR and/or distant metastasis is used to predict or determine the type of adjuvant treatment for the subject following mastectomy and/or breast conserving surgery according to the first aspect of the present invention.

Preferably, the predicted likelihood of LRR and/or distant metastasis is used to predict or determine the type of adjuvant treatment for the subject following mastectomy and/or breast conserving surgery according to the second aspect of the present invention.

Preferably, the predicted likelihood of LRR and/or distant metastasis is used to predict or determine the type of adjuvant treatment for the subject following mastectomy and/or breast conserving surgery according to the third aspect of the present invention.

In accordance with a fourth aspect of the present disclosure, there is provided a method for predicting the likelihood of locoregional recurrence (LRR) and/or distant metastasis in a subject with breast cancer following mastectomy and/or breast conserving surgery, comprising:
i. measuring the expression level of a plurality of genes in a sample isolated from the subject; and
ii. deriving a score based on the measured expression level of the plurality of genes;
wherein the plurality of genes is selected from a group consisting of: TRPV6, DDX39, BUB1B, CCR1, STIL, BLM, C16ORF7, TPX2, PTI1, TCF3, CCNB1, DTX2, PIM1, ENSA, RCHY1, NFATC2IP, OBSL1, MMP15, and a fragment, a homologue, a variant or a derivative thereof; and
wherein the derived score provides an indication of the likelihood of LRR and/or the likelihood of distant metastasis in the subject.

In accordance with a fifth aspect of the present disclosure, there is provided a kit for predicting the likelihood of locoregional recurrence (LRR) and/or distant metastasis in a subject with breast cancer following mastectomy and/or breast conversing surgery, comprising:
i. at least one reagent capable of specifically binding to a plurality of genes in a sample isolated from the subject to quantify the expression level of the plurality of genes; and
ii. a predictive classification model comprising at least one scoring algorithm for deriving a score based on the expression level of the plurality of genes,
wherein the plurality of genes is selected from a group consisting of: TRPV6, DDX39, BUB1B, CCR1, STIL, BLM, C16ORF7, TPX2, PTI1, TCF3, CCNB1, DTX2, PIM1, ENSA, RCHY1, NFATC2IP, OBSL1, MMP15, and a fragment, a homologue, a variant or a derivative thereof; and
wherein the derived score provides an indication of the likelihood of LRR and/or the likelihood of distant metastasis in the subject.

Another aspect of the present disclosure relates to a method for predicting the LRR risk and responses to radiotherapy of breast cancer patients after mastectomy and/or BCS by detecting the expression levels of a gene set consisting of TRPV6, DDX39, BUB1B, CCR1, STIL, BLM, C16ORF7, TPX2, PTI1, TCF3, CCNB1, DTX2, PIM1, ENSA, RCHY1, NFATC2IP, OBSL1, and MMP15. The method comprises: (A) extracting genes (mRNA) from a specimen of a patient after mastectomy and/or BCS; (B) hybridizing the extracted genes with genes in gene probes; (C) each gene probe of the genes is selected from a group consisting of TRPV6, DDX39, BUB1B, CCR1, STIL, BLM, C16ORF7, TPX2, PTI1, TCF3, CCNB1, DTX2, PIM1, ENSA, RCHY1, NFATC2IP, OBSL1, and MMP15; (D) measuring the expression levels of the genes extracted from the specimen; and (E) evaluating an LRR rate of breast cancer using an 18-gene classifier (predictive
classification model) and analysis algorithms for proportional hazards.

In some embodiments, the method of the present disclosure comprises:
getting a tumor tissue sample from a patient after mastectomy or BCS; extracting total RNA from the sample; making the total RNA to contact a gene microarray, which is a gene set consisting of TRPV6, DDX39, BUB1B, CCR1, STIL, BLM, C16ORF7, TPX2, PTI1, TCF3, CCNB1, DTX2, PIM1, ENSA, RCHY1, NFATC2IP, OBSL1, and MMP15; measuring the expression level of one or all of the genes in the gene set; and calculating the hazard ratio using 18-gene scores. In some embodiments, when the score of an algorithm equation applied by the 18-gene classifier is ≥31, the patient is classified into a high-risk group of LRR of breast cancer. When the score is <31, the patient is classified into a low-risk group of LRR of breast cancer.

The disclosure further provides a prediction or estimation method for evaluating the LRR risk and responses to radiotherapy of breast cancer patients after mastectomy, the method comprising: (A) extracting genes (mRNA) from a specimen of a patient after mastectomy and/or BCS; (B) hybridizing the extracted genes with genes in gene probes; (C) each gene probe of the genes is selected from a group consisting of TRPV6, DDX39, BUB1B, CCR1, STIL, BLM, C16ORF7, TPX2, PTI1, TCF3, CCNB1, DTX2, PIM1, ENSA, RCHY1, NFATC2IP, OBSL1, and MMP15; (D) measuring the expression levels of the genes extracted from the specimen; and (E) evaluating an LRR rate of breast cancer using the 18-gene classifier and analysis algorithms for proportional hazards. The method further includes helping to evaluate whether to intervene and treat a patient with radiotherapy when a patient is classified into a high-risk group of LRR of breast cancer so as to prevent LRR of breast cancer.

The disclosure provides a gene chip consisting of 18 genes. When the gene expression level of any of the 4 of the 18 genes (RCHY1, PTI1, ENSA and TRPV6) increases, the LRR risk is reduced. When the gene expression level of any of the other 14 of the 18 genes (BLM, TCF3, PIM1, DDX39, BUB1B, STIL, TPX2, CCNB1, MMP15, CCR1, NFATC2IP, OBSL1, C16ORF7 and DTX2) increases, the LRR risk increases.

Another purpose of the disclosure is to classify patients, wherein when the score of an algorithm equation applied by the classifier is ≥31, the patient is classified into a high-risk group of LRR of breast cancer, and when the score is <31, the patient is classified into a low-risk group of LRR of breast cancer.

Another purpose of the disclosure is to classify patients, wherein when the score of an algorithm equation applied by the classifier is ≥31, the patient is classified into a high-risk group of LRR of breast cancer as well as distant metastasis.

A further purpose of the disclosure is helping to evaluate whether to intervene and treat a patient with radiotherapy when a patient is classified into a high risk group of LRR of breast cancer so as to prevent LRR of breast cancer.

A further purpose of the disclosure is to provide gene probes, which are fixed on a microarray chip.

The measuring of gene expression levels in the evaluation method of the present disclosure includes measuring by quantitative polymerase chain reaction (qPCR), or called reverse transcriptase polymerase chain reaction (RT-PCR).

In the evaluation method of the present disclosure, gene expression levels are analyzed using the 18-gene classifier and analysis algorithms for proportional hazards to evaluate an LRR rate of breast cancer, wherein the analysis algorithms for proportional hazards include algorithms for analysis.

The present disclosure relates to medical equipment for evaluating LRR rates of breast cancer, which employs a method and reading equipment for evaluating LRR rates.

The present disclosure further provides a gene probe set for evaluating an LRR rate of breast cancer, comprising: (A) any gene selected from a group consisting of TRPV6, DDX39, BUB1B, CCR1, STIL, BLM, C16ORF7, TPX2, PTI1, TCF3, CCNB1, DTX2, PIM1, ENSA, RCHY1, NFATC2IP, OBSL1, and MMP15; (B) hybridizing the genes with genes extracted from a breast cancer patient to measure the expression levels of the genes. The genes of the breast cancer patient are analyzed using the 18-gene classifier and analysis algorithms for proportional hazards to evaluate an LRR rate of breast cancer.

The present disclosure also provides a method for predicting the LRR risk and responses to radiotherapy of breast cancer patients using the foregoing gene probe set for evaluating LRR rates of breast cancer.

The present disclosure further provides an evaluation method for evaluating an LRR rate of breast cancer using the foregoing gene probe set, wherein when the score of an algorithm equation applied by the 18-gene classifier is ≥31, the patient is classified into a high-risk group of LRR of breast cancer, so that a precise evaluation message is provided to the personnel concerned to reduce the burden and waste of medical costs, National Health Insurance payments, or insurance resources.

The present disclosure further provides an evaluation method for evaluating an LRR rate of breast cancer using the foregoing gene probe set and using an algorithm equation applied by the 18-gene classifier to precisely predict the risk of organ or lymphoid tissue distant metastasis for a breast cancer patient.

Preferably, the sample specimen is a tumor tissue obtained from a breast cancer tumor and preferably a primary breast cancer tumor tissue. The tissue is analyzed in a conventional method known in the art.

Another aspect of the present disclosure is assessing the LRR risk of a breast cancer patient after mastectomy by determining the expression levels of a gene set in a patient tissue (e.g. breast tumor, other breast tissue, LN tumor and/or tissue and blood) and adjusting the correlation between the patient tissue and an LRR rate of breast cancer. It is found that the gene set highly correlated with LRR rates of breast cancer includes TRPV6, DDX39, BUB1B, CCR1, STIL, BLM, C16ORF7, TPX2, PTI1, TCF3, CCNB1, DTX2, PIM1, ENSA, RCHY1, NFATC2IP, OBSL1, and MMP15.

Gene expression levels may be determined in any method known in the art (e.g. quantitative polymerase chain reaction [qPCR], or called reverse transcriptase polymerase chain reaction [RT-PCR] or a quantitative method to be devised in the future that may provide quantitative information regarding gene expression.

In some embodiments, gene expression profiles are quantitatively determined by gene expression products such as proteins, polypeptides or nucleic acid molecules (e.g. mRNA, tRNA, rRNA). Nucleic acid can be quantitated by the nucleic acid directly or by regular gene sequences. Additionally, gene segments or polymorphic gene segments may also be quantitated.

In a preferred embodiment of the present disclosure, quantitation is achieved by measuring the gene expression levels in a specimen or sample of a breast cancer patient, which may be performed in a conventional method known in the art. In another embodiment, the extent of hybridization of the mRNA in the specimen sample is measured with gene probes fixed on a gene set microarray having a specific suitable nucleic acid. The foregoing microarray is also within the scope of the invention. One method for making an oligonucleotide microarray is described in W.O. Patent No. 95/11995. Other conventional methods already known in the art may also be used.

A gene expression profile may be generated from the initial nucleic acid sample of the specimen using any convenient method. That is, a gene expression profile may be obtained using any conventional method, e.g. analysis method of differential gene expressions already applied in this field. A representative and convenient gene expression profile is generated using microarrays. The evaluation method of the present invention uses a gene microarray to evaluate the genes related to an LRR rate of breast cancer obtained from a patient after BCS, the genes including a gene set consisting of TRPV6, DDX39, BUB1B, CCR1, STIL, BLM, C16ORF7, TPX2, PTI1, TCF3, CCNB1, DTX2, PIM1, ENSA, RCHY1, NFATC2IP, OBSL1, and MMP15. The target nucleic acids of an initially prepared specimen are labeled according to a signal producing system of a sequence. Following the preparation of the target nucleic acids of the specimen sample, the sample of the target nucleic acids is hybridized with a gene array. The hybridized complex of the labeled nucleic acids has a complementary gene sequence to that of the genes on the array surface of the probe. The hybridized complex is detected, either qualitatively or quantitatively.

Gene expression levels are numerically assessed or measured by an apparatus. The numeric values are raw values obtained by the apparatus, and when necessary the numeric values are re-scaled, filtered and/or normalized. The data are obtained, for example, from a gene chip. RTM, probe array or microarray (U.S. Patent Nos. 5,631,734, 5,874,219, 5,861,242, 5,858,659, 5,856,174, 5,843,655, 5,837,832, 5,834,758, 5,770,722, 5,770,456, 5,733,729, and 5,556,752 granted to Affymetrix, Inc., all of which are incorporated herein by reference to their technologies in their entirety). Genome expression levels are calculated in software (e.g. Affymetrix GENECHIP® software). Nucleic acids (e.g. mRNA) obtained from a specimen is hybridized with a probe of a specific microarray (also commonly known as DNA chip or biochip) under stringent conditions.

In some embodiments, the probe kit is fixed on a microarray. Preferably, the microarray is U133 plus 2.0 array.

The data of the raw gene expression levels obtained from the specimen are further assayed, and 18-gene scores are calculated using the proportional hazards model. In an embodiment of the present disclosure, the 18-gene score is calculated using an algorithm with the following equation: Score = TRPV6 + DDX39 + BUB1B + CCR1 + STIL + BLM + C16ORF7 + TPX2 + PTI1 + TCF3 + CCNB1 + DTX2 + PIM1 + ENSA + RCHY1 + NFATC2IP + OBSL1 + MMP15; wherein each gene counts as one point when the hazard ratio is <1, and so the total score = 18.

In another embodiment, the 18-gene score is calculated using an algorithm with the following equation: Score = TRPV6 + DDX39 + BUB1B + CCR1 + STIL + BLM + 2×C16ORF7 + TPX2 + PTI1 + TCF3 + CCNB1 + DTX2 + PIM1 + ENSA + RCHY1 + 2×NFATC2IP + OBSL1 + 2×MMP15; wherein when the hazard ratio is <1, each gene counts as one point if the genes are verified by univariate analysis, each gene counts as two points if the genes are verified by multivariate analysis, and so the total score = 21.

In another embodiment, the 18-gene score is calculated using an algorithm with the following equation in a univariate Cox regression model: Score = 3×TRPV6 + 5× DDX39 + 18×BUB1B + 4×CCR1 + 4×STIL + 7×BLM + 7×C16ORF7 + 4×PMI1 + 9×TPX2 + 8×PTI1 + 3×TCF3 + 7×CCNB1 + 2×DTX2 + 2×ENSA + 5×RCHY1 + 6×NFATC2IP + 2×OBSL1 + 6×MMP15; wherein the score of each gene is re-scaled according to its weighting, and so the total score = 102.

In another embodiment, the 18-gene score is calculated using an algorithm with the following equation in a multivariate Cox regression model: Score = 2×TRPV6 + 2×DDX39 + 2×BUB1B + CCR1 + STIL + 2×BLM + 5×C16ORF7 + 3×PIM1 + 3×TPX2 + 5×PTI1 + TCF3 + CCNB1 + DTX2 + 2×ENSA + 3×RCHY1 + 4×NFATC2IP + OBSL1 + MMP15; wherein the score of each gene is re-scaled according to the odds ratio to a score between 1 and 5, each gene counts as 1 point when the odds ratio is <1, and so the total score = 40.

In another embodiment, the 18-gene score is calculated using an algorithm with the following equation in a univariate Cox regression model: Score = 3×TRPV6 + 5^{×} DDX39 + 18×BUB1B + 4×CCR1 + 4×STIL + 7×BLM + 7×C16ORF7 + 4×PMI1 + 9×TPX2 + 8×PTI1 + 3×TCF3 + 7×CCNB1 + 2×DTX2 + 2×ENSA + 5×RCHY1 + 6×NFATC2IP + 2×OBSL1 + 6×MMP15; wherein the score of each gene is re-scaled according to its weighting, and so the total score = 102.

In another aspect, the present disclosure provides a microarray for evaluating an LRR rate of breast cancer for a patient after mastectomy, the gene set comprising TRPV6, DDX39, BUB1B, CCR1, STIL, BLM, C16ORF7, TPX2, PTI1, TCF3, CCNB1, DTX2, PIM1, ENSA, RCHY1, NFATC2IP, OBSL1, and MMP15.

In a further aspect, the present invention provides a microarray for evaluating an LRR rate of breast cancer for a patient after mastectomy and/or BCS, the gene set comprising TRPV6, DDX39, BUB1B, CCR1, STIL, BLM, C16ORF7, TPX2, PTI1, TCF3, CCNB1, DTX2, PIM1, ENSA, RCHY1, NFATC2IP, OBSL1, and MMP15.

In yet another aspect, the present disclosure provides a gene set to be used in the foregoing evaluation methods. In some embodiments, the gene set includes a reagent for detecting the gene expression of a specimen obtained from a breast cancer patient after mastectomy or BCS, wherein the reagent has any one or more genes from a gene set consisting of the following specific genes being detected: TRPV6, DDX39, BUB1B, CCR1, STIL, BLM, C16ORF7, TPX2, PTI1, TCF3, CCNB1, DTX2, PIM1, ENSA, RCHY1, NFATC2IP, OBSL1, and MMP15.

Other aspects and advantages of the invention will become apparent to those skilled in the art from a review of the ensuing description, which proceeds with reference to the following illustrative drawings of various embodiments of the invention.

### BRIEF DESCRIPTION OF THE DRAWINGS

The present invention will now be described, by way of illustrative example only, with reference to the accompanying drawings, of which:
Figure 1A illustrates a flow chart of an evaluation method according to an embodiment of the present invention for evaluating an LRR rate of breast cancer and risk of distant metastases.
Figure 1B illustrates the 5-year LRR control probabilities for patients whose 18-gene scores are <31 and ≥31.
Figure 2 illustrates a ROC curve of a classifier model according to an embodiment of the present invention.
Figure 3 illustrates the 5-year LRR control probabilities for patients after BCS (94% had adjuvant radiotherapy).
Figure 4: Flow of patient selection and external validation.
Figure 5: Distant Metastasis-Free Probability (DMFP) of Low, Intermediate and High Risk Patients By 18-Gene Classifier (A) All Patients (N=818), (B) Stage I (N=218), (C) Stage II (N=411), (D) Stage III (N=184), (E) Subgroup analysis, (F) Proportion of Low- (Scores of <21), Intermediate- (Scores of 21-43) and High-Risk (Score of ≥44) Patients According to Breast Cancer Subtype (LA: luminal A like; LB: luminal B like; LH: Luminal HER2; TNBC; triple negative breast cancer).
Figure 6: Hazard Ratio Forest Plots In Each Subgroup, 18-Gene Score (as Continuous Variable) in Prediction of (A) Distant Recurrence, (B) Mortality, (C) Local/Regional Recurrence, and (D) Any Recurrence.
Figure 7: (A) Five-year accumulated incidence of LRR in N0-N1 patients without PMRT (n=114). The estimated LRR rate is 5.3 % for patients with scores of 31. (B) The best cut-off is scores of 31, PMRT reduced LRR from 14.9% to 1.6% (p=0.099) and any recurrence from 34.7% to 8.4% (p=0.015).
Figure 8: An unsupervised cluster analysis of 18 genes and supervised clustering in 135 patients according to the 18-gene scores revealed distinct gene expression profiles in patients with and without recurrence. Patients with locoregional recurrence (LRR) are in blue ( 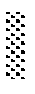 ), both LRR and distant metastasis are in purple ( 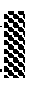 ), distant metastasis patients are in red ( 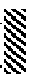 ), and disease free patients are in yellow ( ) as shown at the bottom of the heatmap.

### DETAILED DESCRIPTION

Other features and advantages of the present invention will be further illustrated and described in the following embodiments. The embodiments described herein are only illustrative of the present invention rather than limiting of the present invention.

Unless otherwise defined, technical and scientific terms used herein shall have the meanings that are commonly understood by those of ordinary skill in the art. Furthermore, unless otherwise defined, singular terms shall include pluralities and plural terms shall include the singular. Generally, nomenclatures used in connection with molecular biology, protein, oligonucleotide or polynucleotide chemistry and hybridization technology described herein are those well-known and commonly used in the art. The present invention is not limited to the specific detection methods, detection reagents, and detection devices described herein, for these detection methods and reagents may be moderately modified or changed and achieve the same results and purposes. The scientific terms applied herein are used for concrete description rather than limiting the scope or field of the present invention. Further, unless otherwise required by context, singular terms shall include pluralities and plural terms shall include the singular.

As utilized in accordance with the present disclosure, the following terms, unless otherwise indicated, shall be understood to have the following meanings: The term "and/or" as used herein is to be taken as specific disclosure of each of the two specified features or components with or without the other. For example "A and/or B" is to be taken as specific disclosure of each of (i) A, (ii) B and (iii) A and B, just as if each is set out individually herein.

As used herein, the term "invasive breast cancer" refers to a cancer that spreads outside the membrane of the lobule or duct into the breast tissue. The cancer may then spread into the lymph nodes (LNs) in the armpit or beyond. When breast cancer cells are found in other parts of the body, the cancer is called "metastatic breast cancer".

The term "locoregional recurrence" or "LRR" used herein when related to a breast cancer refers to a recurrence of the disease in the local and/or regional area of the breast which includes areas in the breast, chest wall, axillary, infraclavicular, supraclavicular or parasternal lymph node (LN) area after treatment with mastectomy and/or BCS.

The term "distant metastasis" used herein refers to breast cancer that has spread from the original (primary) tumor to one or more other parts of the body, organs or distant lymph nodes (lymph nodes that are not covered under the term "LRR" as described in the above paragraph) following mastectomy and/or BCS.

The term "multivariate statistics" refers to a form of statistics encompassing the simultaneous observation and analysis of more than one outcome variable. The use of multivariate statistics is called "multivariate analysis".

As used herein, the term "proportional hazards model" refers to a survival model in statistics wherein when the survival data further includes covariates and risk factors, they may be used to estimate the effect of these covariates on the survival time and to predict the survival chance within a specific period of time. The Cox proportional hazards model was proposed by Sir. David Cox in 1972, which is a regressive analysis model most commonly used in survival analysis. This method is often called the Cox model or the proportional hazards model.

As used herein, the term "hybridization" of nucleic acids refers to a process of joining two complementary strands of nucleic acids, such as RNA and DNA, or oligonucleotides. In some embodiments, nucleic acid molecules and their corresponding sense strands of genes are verified simultaneously or in sequential order, or nucleic acid molecules are complementary to their corresponding sense strands. Typically, nucleic acid molecules hybridize with the sense strands under stringent conditions and present the degree of their correspondence.

As used herein, the term "microarray" refers to a collection of microscopic DNA spots aggregated and attached to a solid surface. Each DNA spot contains a picomole of a specific DNA sequence, known as a probe. These can be a short section of a gene or a DNA unit that is hybridized with a cDNA or cRNA (also called anti-sense RNA) in the specimen (called target) sample under high-stringency conditions.

The term "stringency" refers to the extent to which hybridization may occur between nucleic acids with mismatched sequences. High-stringency conditions require absolute complementarity between the molecules, while low-stringency conditions permit hybridization when there are some mismatched bases. Typically, high-stringency conditions are achieved either by reducing NaCl concentration or increasing temperature up to approaching the melt temperature (Tm) of the molecules involved. An example of high-stringency conditions is hybridization at 50°C or higher (e.g. 55°C) and at 0.1 SSC (0.15M NaCl and 0.015M sodium citrate).

As used herein, the term "plurality of genes" refers to two genes or more than two genes.

As used here, the abbreviation "DMFP" refers to probability of freedom from distant metastasis. Distant metastasis here is defined as biopsy confirmed or clinically diagnosed as recurrent invasive breast cancer.

As used herein, the abbreviation "HER2" refers to human epidermal growth factor receptor type 2.

As used herein, the abbreviation "LRCP" refers to local/regional control probability, the probability of freedom from LRR.

As used herein, the abbreviation "LVI" refers to lymphovascular invasion.

As used herein, the abbreviation "OS" refers to overall survival, which is the death attributed to any cause, including breast cancer, non-breast cancer, or unknown cause.

As used herein, the abbreviation "PMRT" refers to post-mastectomy radiotherapy.

As used herein, the abbreviation "RFP" refers to relapse-free probability. Relapse here is defined as any LRR and/or distant metastasis.

A nucleic acid or fragment thereof is "substantially homologous" ("or substantially similar") to another if, when optimally aligned (with appropriate nucleotide insertions or deletions) with the other nucleic acid (or its complementary strand), there is nucleotide sequence identity in at least about 60% of the nucleotide bases, usually at least about 70%, more usually at least about 80%, preferably at least about 90%, and more preferably at least about 95-98% of the nucleotide bases.

Alternatively, substantial homology or (identity) exists when a nucleic acid or fragment thereof will hybridize to another nucleic acid (or a complementary strand thereof) under selective hybridization conditions, to a strand, or to its complement. Selectivity of hybridization exists when hybridization that is substantially more selective than total lack of specificity occurs. Typically, selective hybridization will occur when there is at least about 55% identity over a stretch of at least about 14 nucleotides, preferably at least about 65%, more preferably at least about 75%, and most preferably at least about 90%. The length of homology comparison, as described, may be over longer stretches, and in certain embodiments will often be over a stretch of at least about nine nucleotides, usually at least about 20 nucleotides, more usually at least about 24 nucleotides, typically at least about 28 nucleotides, more typically at least about 32 nucleotides, and preferably at least about 36 or more nucleotides.

Thus, polynucleotides of the disclosure preferably have at least 75%, more preferably at least 85%, more preferably at least 90% homology to the sequences shown in List 1 or the sequence listings herein. More preferably there is at least 95%, more preferably at least 98%, homology. Nucleotide homology comparisons may be conducted as described below for polypeptides. A preferred sequence comparison program is the GCG Wisconsin Best fit program described below. The default scoring matrix has a match value of 10 for each identical nucleotide and -9 for each mismatch. The default gap creation penalty is -50 and the default gap extension penalty is -3 for each nucleotide.

In the context of the present disclosure, a homologue or homologous sequence is taken to include a nucleotide sequence which is at least 60, 70, 80 or 90% identical, preferably at least 95 or 98% identical at the amino acid level over at least 20, 50, 100, 200, 300, 500 or 1000 nucleotides with the nucleotides sequences set out in the sequence listings or in List 1 below. In particular, homology should typically be considered with respect to those regions of the sequence that encode contiguous amino acid sequences known to be essential for the function of the protein rather than non-essential neighbouring sequences. Preferred polypeptides of the invention comprise a contiguous sequence having greater than 50, 60 or 70% homology, more preferably greater than 80, 90, 95 or 97% homology, to one or more of the nucleotides sequences set out in the sequences. Preferred polynucleotides may alternatively or in addition comprise a contiguous sequence having greater than 80, 90, 95 or 97% homology to the sequences set out in the sequence listings or in List 1 below that encode polypeptides comprising the corresponding amino acid sequences.

Other preferred polynucleotides comprise a contiguous sequence having greater than 40, 50, 60, or 70% homology, more preferably greater than 80, 90, 95 or 97% homology to the sequences set out that encode polypeptides comprising the corresponding amino acid sequences.

Nucleotide sequences are preferably at least 15 nucleotides in length, more preferably at least 20, 30, 40, 50, 100 or 200 nucleotides in length.

Generally, the shorter the length of the polynucleotide, the greater the homology required to obtain selective hybridization. Consequently, where a polynucleotide of the invention consists of less than about 30 nucleotides, it is preferred that the % identity is greater than 75%, preferably greater than 90% or 95% compared with the nucleotide sequences set out in the sequence listings herein or in List 1 below. Conversely, where a polynucleotide of the invention consists of, for example, greater than 50 or 100 nucleotides, the % identity compared with the sequences set out in the sequence listings herein or List 1 below may be lower, for example greater than 50%, preferably greater than 60 or 75%.

The "polynucleotide" compositions of this invention include RNA, cDNA, genomic DNA, synthetic forms, and mixed polymers, both sense and antisense strands, and may be chemically or biochemically modified or may contain non-natural or derivatized nucleotide bases, as will be readily appreciated by those skilled in the art. Such modifications include, for example, labels, methylation, substitution of one or more of the naturally occurring nucleotides with an analog, internucleotide modifications such as uncharged linkages (e.g., methyl phosphonates, phosphotriesters, phosphoamidates, carbamates, etc.), charged linkages (e.g., phosphorothioates, phosphorodithioates, etc.), pendent moieties (e.g., polypeptides), intercalators (e.g., acridine, psoralen, etc.), chelators, alkylators, and modified linkages (e.g., alpha anomeric nucleic acids, etc.). Also included are synthetic molecules that mimic polynucleotides in their ability to bind to a designated sequence via hydrogen bonding and other chemical interactions. Such molecules are known in the art and include, for example, those in which peptide linkages substitute for phosphate linkages in the backbone of the molecule.

The term "polypeptide" refers to a polymer of amino acids and its equivalent and does not refer to a specific length of the product; thus, peptides, oligopeptides and proteins are included within the definition of a polypeptide. This term also does not refer to, or exclude modifications of the polypeptide, for example, glycosylations, acetylations, phosphorylations, and the like. Included within the definition are, for example, polypeptides containing one or more analogs of an amino acid (including, for example, natural amino acids, etc.), polypeptides with substituted linkages as well as other modifications known in the art, both naturally and non-naturally occurring.

In the context of the present disclosure, a homologous sequence is taken to include an amino acid sequence which is at least 60, 70, 80 or 90% identical, preferably at least 95 or 98% identical at the amino acid level over at least 20, 50, 100, 200, 300 or 400 amino acids with the sequences set out in the sequence listings or in List 1 below that encode polypeptides comprising the corresponding amino acid sequences. In particular, homology should typically be considered with respect to those regions of the sequence known to be essential for the function of the protein rather than non-essential neighbouring sequences. Preferred polypeptides of the invention comprise a contiguous sequence having greater than 50, 60 or 70% homology, more preferably greater than 80 or 90% homology, to one or more of the corresponding amino acids.

Other preferred polypeptides comprise a contiguous sequence having greater than 40, 50, 60, or 70% homology, of the sequences set out in the sequence listings or in List 1 below that encode polypeptides comprising the corresponding amino acid sequences. Although homology can also be considered in terms of similarity (i.e. amino acid residues having similar chemical properties/functions), in the context of the present disclosure it is preferred to express homology in terms of sequence identity. The terms "substantial homology" or "substantial identity", when referring to polypeptides, indicate that the polypeptide or protein in question exhibits at least about 70% identity with an entire naturally-occurring protein or a portion thereof, usually at least about 80% identity, and preferably at least about 90 or 95% identity.

Homology comparisons can be conducted by eye, or more usually, with the aid of readily available sequence comparison programs. These commercially available computer programs can calculate % homology between two or more sequences.

Percentage (%) homology may be calculated over contiguous sequences, i.e. one sequence is aligned with the other sequence and each amino acid in one sequence directly compared with the corresponding amino acid in the other sequence, one residue at a time. This is called an "ungapped" alignment. Typically, such ungapped alignments are performed only over a relatively short number of residues (for example less than 50 contiguous amino acids).

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example when using the GCG Wisconsin Best fit package (see below) the default gap penalty for amino acid sequences is -12 for a gap and -4 for each extension.

Calculation of maximum % homology therefore firstly requires the production of an optimal alignment, taking into consideration gap penalties. A suitable computer program for carrying out such an alignment is the GCG Wisconsin Best fit package (University of Wisconsin, U.S.A.; Devereux et al., 1984, Nucleic Acids Research 12:387). Examples of other software that can perform sequence comparisons include, but are not limited to, the BLAST package (see Ausubel et al., 1999 ibid - Chapter 18), FASTA (Atschul et al., 1990, J. Mol. Biol., 403-410) and the GENEWORKS suite of comparison tools. Both BLAST and FASTA are available for offline and online searching (see Ausubel et al., 1999 ibid, pages 7-58 to 7-60). However it is preferred to use the GCG Bestfit program.

Although the final % homology can be measured in terms of identity, the alignment process itself is typically not based on an all-or-nothing pair comparison. Instead, a scaled similarity score matrix is generally used that assigns scores to each pair-wise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see user manual for further details). It is preferred to use the public default values for the GCG package, or in the case of other software, the default matrix, such as BLOSUM62.

Once the software has produced an optimal alignment, it is possible to calculate % homology, preferably % sequence identity. The software typically does this as part of the sequence comparison and generates a numerical result.

A polypeptide "fragment," "portion" or "segment" is a stretch of amino acid residues of at least about five to seven contiguous amino acids, often at least about seven to nine contiguous amino acids, typically at least about nine to 13 contiguous amino acids and, most preferably, at least about 20 to 30 or more contiguous amino acids.

Preferred polypeptides of the disclosure have substantially similar function to the sequences set out in the sequence listings or in List 1 below. Preferred polynucleotides of the disclosure encode polypeptides having substantially similar function to the sequences set out in the sequence listings or in List 1 below. "Substantially similar function" refers to the function of a nucleic acid or polypeptide homologue, variant, derivative or fragment of the sequences set out in the sequence listings or in **List 1** below that encodes polypeptides comprising corresponding amino acid sequences.

Nucleic acid hybridization will be affected by such conditions as salt concentration, temperature, or organic solvents, in addition to the base composition, length of the complementary strands, and the number of nucleotide base mismatches between the hybridizing nucleic acids, as will be readily appreciated by those skilled in the art. Stringent temperature conditions will generally include temperatures in excess of 30 degrees Celsius, typically in excess of 37 degrees Celsius, and preferably in excess of 45 degrees Celsius. Stringent salt conditions will ordinarily be less than 1000 mM, typically less than 500 mM, and preferably less than 200 mM. However, the combination of parameters is much more important than the measure of any single parameter. An example of stringent hybridization conditions is 65 degree Celsius and 0.1xSSC (1xSSC = 0.15 M NaCl, 0.015 M sodium citrate pH 7.0).

Other features and advantages of the present invention will be illustrated and described in the following examples or embodiments. The examples or embodiments described herein are only illustrative examples of the present invention and do not limit the present invention in any way whatsoever.

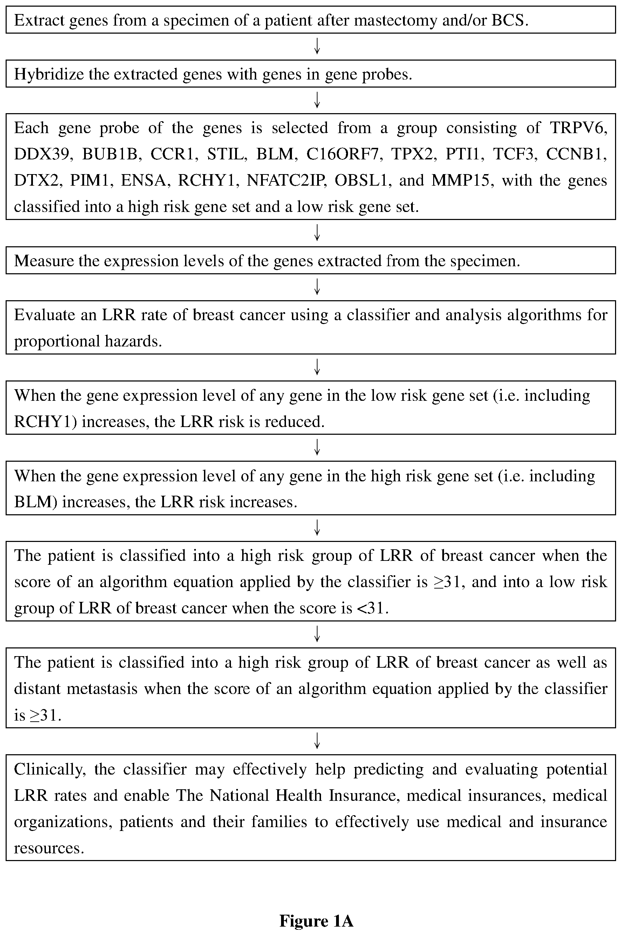

Figure 1A illustrates a flow chart of an evaluation method according to an embodiment of the present invention for evaluating a locoregional recurrence (LRR) rate of breast cancer and risk of distant metastases.

In accordance with an embodiment of the present disclosure, there is described a method for predicting the likelihood of locoregional recurrence (LRR) and/or distant metastasis in a subject with breast cancer following mastectomy and/or breast conserving surgery, comprising measuring the expression level of at least one gene in a sample isolated from the subject; and deriving a score based on the measured expression level of the at least one gene; wherein the at least one gene is selected from a group consisting of: TRPV6, DDX39, BUB1B, CCR1, STIL, BLM, C16ORF7, TPX2, PTI1, TCF3, CCNB1, DTX2, PIM1, ENSA, RCHY1, NFATC2IP, OBSL1, MMP15, and a fragment, a homologue, a variant or a derivative thereof; and wherein the derived score provides an indication of the likelihood of LRR and/or the likelihood of distant metastasis in the subject.

The at least one gene which is selected from the group of 18 genes mentioned above comprises (a) a polynucleotide comprising a nucleotide sequence set forth in any one of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, or a fragment, a homologue, a variant or a derivative thereof; (b) a polynucleotide comprising a nucleotide sequence set forth in any one of the sequences of (a), that encodes a polypeptide comprising the corresponding amino acid sequence; or (c) a polynucleotide comprising a nucleotide sequence capable of hybridising selectively to any one of the sequences of (a), (b), or a complement thereof.

A predictive classification model, which comprises at least one scoring algorithm, is used to perform the step of deriving a score based on the measured expression level of the at least one gene.

When the derived score is less than a first pre-determined reference, the subject is classified into a low risk group of LRR and/or distant metastasis. When the derived score is equal to or more than the first pre-determined reference, the subject is classified into a high risk group of LRR and/or distant metastasis.

When the derived score is less than a second pre-determined reference, the subject is classified into a low risk group of distant metastasis and/or LRR. When the derived score is equal to or more than a third pre-determined reference, the subject is classified into a high risk group of distant metastasis and/or LRR. When the derived score is between the second pre-determined reference (inclusive) and the third pre-determined reference, the subject is classified into an intermediate risk group of distant metastasis and/or LRR.

Preferably, the first pre-determined reference is a score of 31, the second pre-determined reference is a score of 21, and the third pre-determined reference is a score of 44.

In accordance with another embodiment of the present disclosure, there is described a kit for predicting the likelihood of LRR and/or distant metastasis in a subject with breast cancer following mastectomy and/or breast conversing surgery, comprising at least one reagent capable of specifically binding to at least one gene in a sample isolated from the subject to quantify the expression level of the at least one gene; and a predictive classification model comprising at least one scoring algorithm for deriving a score based on the expression level of the at least one gene, wherein the at least one gene is selected from a group consisting of: TRPV6, DDX39, BUB1B, CCR1, STIL, BLM, C16ORF7, TPX2, PTI1, TCF3, CCNB1, DTX2, PIM1, ENSA, RCHY1, NFATC2IP, OBSL1, MMP15, and a fragment, a homologue, a variant or a derivative thereof; and wherein the derived score provides an indication of the likelihood of LRR and/or the likelihood of distant metastasis in the subject.

The at least one gene which is selected from the group of 18 genes mentioned above comprises (a) a polynucleotide comprising a nucleotide sequence set forth in any one of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, or a fragment, a homologue, a variant or a derivative thereof; (b) a polynucleotide comprising a nucleotide sequence set forth in any one of the sequences of (a), that encodes a polypeptide comprising the corresponding amino acid sequence; or (c) a polynucleotide comprising a nucleotide sequence capable of hybridising selectively to any one of the sequences of (a), (b), or a complement thereof.

In accordance with another embodiment of the present disclosure there is described a microarray for predicting the likelihood of LRR and/or distant metastasis in a subject with breast cancer following mastectomy and/or breast conserving surgery, comprising at least one gene probe for measuring the expression level of at least one gene selected from a group consisting of: TRPV6, DDX39, BUB1B, CCR1, STIL, BLM, C16ORF7, TPX2, PTI1, TCF3, CCNB1, DTX2, PIM1, ENSA, RCHY1, NFATC2IP, OBSL1, MMP15, and a fragment, a homologue, a variant or a derivative thereof.

The at least one gene which is selected from the group of 18 genes mentioned above comprises (a) a polynucleotide comprising a nucleotide sequence set forth in any one of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, or a fragment, a homologue, a variant or a derivative thereof; (b) a polynucleotide comprising a nucleotide sequence set forth in any one of the sequences of (a), that encodes a polypeptide comprising the corresponding amino acid sequence; or (c) a polynucleotide comprising a nucleotide sequence capable of hybridising selectively to any one of the sequences of (a), (b), or a complement thereof.

Surprisingly, and advantageously, any number of the 18 genes of the present invention mentioned in the various embodiments above, and in any combination, can be used for predicting the likelihood or LRR and/or distant metastasis in a subject with breast cancer following mastectomy and/or breast conserving surgery.

Furthermore, and advantageously, the predicted likelihood of LRR and/or distant metastasis can be used to predict or determine the type of adjuvant treatment for the subject following mastectomy and/or breast conserving surgery.

### EXAMPLES

### Example 1

### DNA microarray analysis for verifying gene expression profiles related to locoregional recurrence rates after mastectomy or breast conversing surgery

Patients with invasive breast cancer were shortlisted in a medical research of tumor genes to develop a new taxonomy of breast cancer. A total of 217 patients with invasive breast cancer who underwent mastectomy or breast conserving surgery (BCS) between 2005 and 2012 and who had tissue specimens available for DNA (or gene) microarray were selected for this study. Of the 217 patients, 130 patients underwent mastectomy and 87 patients underwent BCS. All of the 217 patients gave their consent to have their primary tumor tissues undergo a study of DNA (or gene) microarray. Patients eligible for the study should have no post mastectomy radiotherapy (PMRT) (n=130) within a minimum of two years of follow-up. Patients who had accepted breast conserving surgery (BCS) (n=87) were analyzed separately to examine the performance of gene expression profiling in the prediction of locoregional recurrence (LRR) rates.

Clinical characteristics of patients after mastectomy or BCS in the study are shown in Table 1 below. Based on the clinical characteristics, majority of the patients were in stage T2 or higher (56%, 73/130), and 93% (121/130) had N0 and N1 diseases. No patients had received radiotherapy after mastectomy or BCS.

**Table 1: Clinical characteristics of patients after mastectomy or BCS**

| Characteristics | Mastectomy (n=130) | BCS (n=87) | Chi-square P-value |
|---|---|---|---|
| Median follow-up (months) | 61.9 | 60.1 | |
| Age | | | 0.7433 |
| ≤40 | 26 | 19 | |
| >40 | 104 | 68 | |
| T stage | | | 0.0256 |
| T1 | 57 | 54 | |
| T2 | 72 | 33 | |
| >T2 | 1 | 0 | |
| Number of axillary lymph node (LN) invasions | | | 0.5982 |
| N0 | 92 | 56 | |
| N1-3 | 29 | 23 | |
| N≥4 | 9 | 8 | |
| ER status | | | 0.2554 |
| Negative | 47 | 25 | |
| Positive | 83 | 62 | |
| Progesterone receptor status (PR status) | | | 0.0183 |
| Negative | 69 | 32 | |
| Positive | 61 | 55 | |
| HER2 overexpression | | | 0.1783 |
| Negative | 79 | 62 | |
| Indeterminate | 2 | 0 | |
| Positive | 49 | 25 | |
| Lymphovascular (LV) invasion | | | 0.5551 |
| Absent /Focal | 105 | 73 | |
| Prominent | 25 | 14 | |
| Nuclear grade | | | 0.6421 |
| Grade 1 | 22 | 12 | |
| Grade 2 | 38 | 30 | |
| Grade 3 | 70 | 44 | |
| Adjuvant chemotherapy | | | 0.0117 |
| No | 35 | 11 | |
| Yes | 95 | 76 | |
| Adjuvant radiotherapy | | | <0.0001 |
| No | 130 | 5 | |
| Yes | 0 | 82 | |
| Adjuvant hormonal therapy | | | 0.0637 |
| No | 55 | 26 | |
| Yes | 75 | 61 | |

The frozen tissue samples from each of the 217 patients were obtained from surgical specimens of primary tumors taken from the patients prior to treatment (mastectomy or BCS). Total RNA was extracted from tumor tissues with Trizol (Invitrogen, Carlsbad, CA) and purified with an RNeasy Mini Kit (Qiagen, Valencia, CA), and qualitatively assessed by an Agilent 2100 Bioanalyzer. Hybridization targets were prepared from total RNA according to the Affymetrix protocol and hybridized to U133 plus 2.0 arrays.

18 genes were found to be significantly related to LRR rates in the 130 mastectomy patients after univariate analysis of the Cox proportional hazards model. The functions of these 18 genes are associated with genes that are involved in oncogenic process, proliferation, invasion, inflammation, cell-cell interaction, apoptosis, and metabolism. Among these genes that are most significantly related to LRR rates, BLM, TCF3, PIM1, RCHY1, and PTI1 are involved in oncogenic process; DDX39, BUB1B, STIL, TPX2, and CCNB1 are involved in proliferation; MMP15 is associated with invasion; CCR1 and NFATC21P are involved in inflammation; TRPV6 and DTX2 are associated with cell death; and ENSA is correlated with metabolism (see Table 2 below). See also Table 3 which lists the 18 genes in a 22 probe set.

**Table 2: Processes or pathways that each of the 18 genes related to LRR rates is involved in**

| |
|---|
| • Oncogenic process: BLM, TCF3, PIM1, RCHY1*, and PTI1* |
| • Proliferation: DDX39, BUB 1B, STIL, TPX2, and CCNB 1 |
| • Invasion: MMP15 |
| • Inflammation: TRPV6 and DTX2 |
| • Cell-cell interaction: TRPV6* and OBSL1 |
| • Cell death: TRPV6 and DTX2 |
| • Metabolism: ENSA* |
| * Over expression of these genes correlate to lower LRR rates |

**Table 3: The 18 genes in a 22 probe set - The optimal cut-off point**

| COL | Gene Probe Set | Gene | Cut-off point | High risk | Low risk |
|---|---|---|---|---|---|
| COL4 | 1559405_a_at | TRPV6 | 3.96 | <3.96 | ≥3.96 |
| COL9 | 201584_s_at | DDX39 | 9.43 | ≥9.43 | <9.43 |
| COL17 | 203755_at | BUB1B | 7.5 | ≥7.5 | <7.5 |
| COL20 | 205099_s_at | CCR1 | 6.4 | ≥6.4 | <6.4 |
| COL21 | 205339_at | STIL | 6.7 | ≥6.7 | <6.7 |
| COL23 | 205733_at | BLM | 6.1 | ≥6.1 | <6.1 |
| COL24 | 205781_at | C16ORF7 | 6.18 | ≥6.18 | <6.18 |
| COL33 | 209193_at | PIM1 | 7.25 | ≥7.25 | <7.25 |
| COL35 | 210052_s_at | TPX2 | 7.4 | ≥7.4 | <7.4 |
| COL41 | 212743_at | RCHY1 | 6.12 | <6.12 | ≥6.12 |
| COL43 | 212775_at | OBSL1 | 8.25 | <8.25 | ≥8.25 |
| COL45 | 212808_at | NFATC2IP | 7.1 | ≥7.1 | <7.1 |
| COL48 | 213477_x_at | PTI1 | 13.85 | <13.85 | ≥13.85 |
| COL52 | 213809_x_at | TCF3 | 5.69 | ≥5.69 | <5.69 |
| COL57 | 214710_s_at | CCNB1 | 7.83 | ≥7.83 | <7.83 |
| COL60 | 215732_s_at | DTX2 | 6.4 | ≥6.4 | <6.4 |
| COL75 | 228729_at | PIM1 | 6.9 | ≥6.9 | <6.9 |
| COL76 | 228851_s_at | ENSA | 8.61 | <8.61 | ≥8.61 |
| COL80 | 235300_x_at | RCHY1 | 5.64 | <5.64 | ≥5.64 |
| COL82 | 238130_at | NFATC2IP | 6.25 | ≥6.25 | <6.25 |
| COL83 | 238776_x_at | OBSL1 | 5.34 | ≥5.34 | <5.34 |
| COL84 | 243883_at | MMP15 | 5.93 | ≥5.93 | <5.93 |

### Example 2

### Gene expression profiles of LRR rates verified by statistical analysis

The 34 genes in the new platform of this invention were distributed in an 84 gene probe set, and 4 genes with unknown functions were unable to be identified. After univariate analysis, 18 out of the remaining 30 genes were found to be able to classify mastectomy patients into a low risk and a high risk group based on LRR rates. After multivariate analysis, patients may be classified into a low risk group and a high risk group. The 18 genes were used to classify BCS patients into a low risk group and a high risk group. Patients whose 18-gene scores were ≥31 were defined as at high risk and those whose scores were <31 were defined as at low risk.

### Algorithms of scoring based on the 18 genes (18-gene panel or classifier)

Table 4 shows the univariate analysis and multivariate analysis of deriving or calculating scores based on each of the 18 genes using the proportional hazards model. When the hazard ratio is <1, it counts as one in multivariate analysis.

**Table 4: Univariate analysis and multivariate analysis using proportional hazards model**

| Parameter | Single Hazard Ratio | Multiple Hazard Ratio | Pr > ChiSq |
|---|---|---|---|
| TRPV6 | 2.769 | 2.48 | 0.1604 |
| DDX39 | 5.401 | 2.123 | 0.3525 |
| BUB1B | 17.783 | 3.291 | 0.4061 |
| CCR1 | 3.585 | 0.941 | 0.9378 |
| STIL | 3.965 | 1.215 | 0.8093 |
| BLM | 7.160 | 1.999 | 0.4305 |
| C16ORF7 | 7.027 | 4.891 | 0.0494 |
| TPX2 | 8.580 | 3.141 | 0.5113 |
| PTI1 | 8.349 | 3.745 | 0.0853 |
| TCF3 | 3.125 | 1.384 | 0.5892 |
| CCNB1 | 6.659 | 0.323 | 0.5195 |
| DTX2 | 2.233 | 0.885 | 0.8258 |
| PIM1 | 5.665 | 0.614 | 0.5861 |
| ENSA | 1.900 | 1.924 | 0.254 |
| RCHY1 | 5.239 | 3.48 | 0.0406 |
| NFATC2IP | 5.492 | 3.506 | 0.0797 |
| OBSL1 | 2.300 | 1.403 | 0.5516 |
| MMP15 | 6.072 | 1.377 | 0.6519 |

Below are four exemplary algorithms of scoring based on the 18 gene panel:
**Algorithm 1:** One point for each gene, and there are 18 genes in total. Total score = 18. Score = TRPV6 + DDX39 + BUB1B + CCR1 + STIL + BLM + C16ORF7 + TPX2 + PTI1 + TCF3 + CCNB1 + DTX2 + PIM1 + ENSA + RCHY1 + NFATC2IP + OBSL1 + MMP15

| Risk | Number of patients without LRR | Number of LRRs | LRR rate | Total number of patients | Chi-square P-value |
|---|---|---|---|---|---|
| Low (<13) | 93 | 2 | 2.1% | 95 | <0.0001 |
| High (≥13) | 24 | 11 | 31.4% | 35 | |

**Algorithm 2:** When the hazard ratio is <1, each gene counts as one point if the genes are verified by univariate analysis, each gene counts as two points if the genes are verified by multivariate analysis, and so the total score = 21.
Score = TRPV6 + DDX39 + BUB1B + CCR1 + STIL + BLM + 2×C16ORF7 + TPX2 + PTI1 + TCF3 + CCNB1 + DTX2 + PIM1 + ENSA + RCHY1 + 2×NFATC2IP + OBSL1 + 2×MMP15

| Relapse Index | | | | |
|---|---|---|---|---|
| Index | Relapse | | | |
| | No relapse | Relapse | Total | P-value |
| <15 | 95 | 2 | 97 | <0.0001 |
| | 97.9% | 2.1% | | |
| ≥15 | 22 | 11 | 33 | |
| | 66.7% | 33.3% | | |

**Algorithm 3:** The 18-gene score is calculated based on univariate odds ratio of univariate analysis. The score of each gene is re-scaled according to its weighting, and so the total score = 102.
Score = 3×TRPV6 + 5×DDX39 + 18×BUB1B + 4×CCR1 + 4×STIL + 7×BLM + 7×C16ORF7 + 4×PIM1 + 9×TPX2 + 8×PTI1 + 3×TCF3 + 7×CCNB1 + 2×DTX2 + 2×ENSA + 5×RCHY1 + 6×NFATC2IP + 2×OBSL1 + 6×MMP15

| Relapse Index | | | | |
|---|---|---|---|---|
| Index | Relapse | | | |
| | No relapse | Relapse | Total | P-value |
| <83 | 100 | 2 | 102 | <0.0001 |
| | 98.0 | 2.0% | | |
| ≥83 | 17 | 11 | 28 | |
| | 60.7% | 39.3% | | |

**Algorithm 4:** The 18-gene score is calculated based on odds ratio of multivariate analysis. The score of each gene is re-scaled according to the odds ratio to a score between 1 and 5; each gene counts as 1 point when the odds ratio is <1, and so the total score = 40.
Score = 2×TRPV6 + 2×DDX39 + 2×BUB1B + CCR1 + STIL + 2×BLM + 5×C16ORF7 + 3×PIM1 + 3×TPX2 + 5×PTI1 + TCF3 + CCNB1 + DTX2 + 2×ENSA + 3× RCHY1 + 4×NFATC2IP + OBSL1 + MMP15

| Score | Relapse | | | |
|---|---|---|---|---|
| | No relapse | Relapse | Total | P-value |
| <31 | 100 | 1 | 101 | <0.0001 |
| | 99.0% | 1.0% | | |
| ≥31 | 17 | 12 | 35 | |
| | 58.6% | 41.4% | | |

### Results

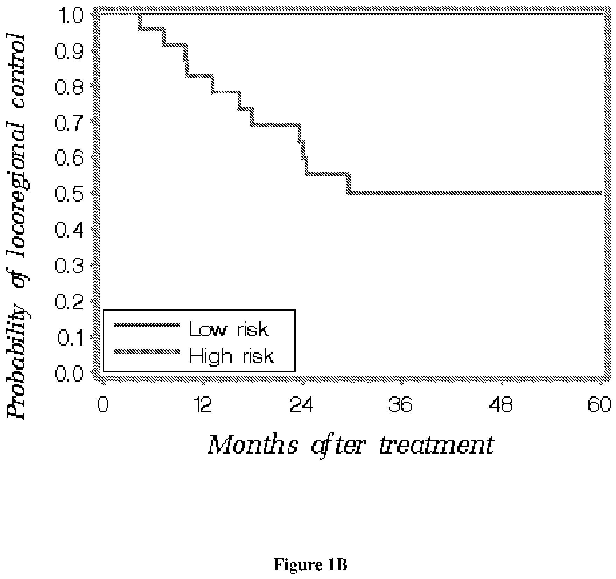

Clinical decisions are intended to be philosophically more conservative and tend toward over-treating patients. On that basis the optimal cut-off value (or pre-determined reference) was score 31 on the Receiver Operating Characteristic (ROC) curve. The overall accuracy of these predictions was 87%, with an estimated sensitivity of 91% and a specificity of 87%. The 5-year locoregional control probabilities in patients whose 18-gene scores were ≥31 and <31 was 50% and 100% (p<0.0001), respectively (see Figure 1B).

Lymph node (LN) status is an important factor in the overall diagnosis. It may be used to determine whether a primary tumor has been spread in a way known as "distant metastasis". It may be used for evaluation by calculation and may provide references for subsequent treatments.

According to LN status, in N0 and N1 patients whose 18-gene scores were ≥31 and <31 respectively, the 5-year LR control probabilities were statistically different (50% versus 100%, p<0.0001). The number of N2 patients was too small to draw a conclusion, but the predictive power of the 18-gene panel was similar to that of N0-N1 patients. Patients defined by the 18-gene classifier (or predictive classification model) as high risk had very low 5-year metastasis-free survival and overall survival rates, with or without LN metastasis. Further details and data are provided in Table 4 above.

**Table 5: Mastectomy patients were classified into subgroups with different degrees of risk by the 18 gene panel according to their LN status**

| 18-gene classifier | Number of Patients | Five-year LR control probability | Five-year metastasis- free probability | Five-year overall survival probability |
|---|---|---|---|---|
| N0 patients | 92 | | | |
| Score ≥31 | 8 | 50.0% | 37.5% | 50.0% |
| Score <31 | 84 | 100.0% | 92.8% | 94.4% |
| P-value | | <0.0001 | <0.0001 | <0.0001 |
| N1 patients | 29 | | | |
| Score ≥31 | 11 | 50.0% | 20.0% | 23.6% |
| Score <31 | 18 | 100.0% | 87.7% | 83.0% |
| P-value | | 0.0032 | 0.0017 | 0.0071 |
| ≥N2 patients | 9 | | | |
| Score ≥31 | 5 | 60.0% | 0.0% | 0.0% |
| Score <31 | 4 | 100.0% | 25.0% | 50.0% |
| P-value | | 0.1803 | 0.2539 | 0.2701 |

As Table 5 shows, the profiling based on the 18 gene panel was mainly for N0 and N1 mastectomy patients. In the present study, the LRR rate for N0 patients was about 5%; our gene classifier (18 gene panel) confirmed that 9% of N0 patients were at high risk, 50% of whom would have cancer relapse. By contrast, the LRR rate for N1 patients was about 20%; our gene classifier (18 gene panel) confirms that 38% of N1 patients were at high risk, 50% of whom will have cancer relapse. As for N2 patients, the number of our samples was too small for us to draw any conclusion, but the prediction accuracy was similar; 55% of N2 patients would be classified as at high risk.

The consistency of the performance of the 18-gene classifier in both mastectomy patients and BCS patients is very positive. This strongly suggests that the 18-gene classifier can predict whether breast cancer patients are at risk of LRR.

### Example 3

### The Cox proportional hazards model of mastectomy patients

Based on current clinical practice, methods used for evaluating whether N1 breast cancer patients require adjuvant radiotherapy would assign adjuvant radiotherapy to around 80% of them. However, radiotherapy reduces LRR rates for these patients, prevents secondary distant metastasis caused by relapses, and improves the overall survival rate.

The Cox proportional hazards model had been widely used to describe survival rates and related variables. Subsequently, the study examined whether the (18 gene panel) classifier was an independent prognostic factor that was related to LRR rate control. Traditional proportional hazards analysis had established clinical parameters that were related to proportional hazards analysis and quantitative evaluation, including the extent of LN metastasis and the ER status related to LRR. We employed clinical parameters and the classification system of the classifier to fully analyze the proportional hazards of the specimens obtained from the 130 patients (who underwent mastectomy).

It was confirmed that a combination of these traditional clinical variables with the (18 gene panel) classifier may be a significant independent factor for predicting LRR rates. The use of the (18 gene panel) classifier and the proportional hazards analysis showed that when the 18-gene score of a patient was ≥31 (see Table 6), the hazard ratio of LRR rates was 67.8 (95% confidence interval, 8.3-552.5). In the present study, it was again confirmed that N0 and N1 patients may be classified into more homogeneous subgroups using a novel gene expression profile and evaluation model.

**Table 6: Cox proportional hazards model of all mastectomy patients**

| Variable | | Hazard ratio (95% confidence interval) of LRR rate | P-value |
|---|---|---|---|
| Without the 18-gene classifier analysis | | | |
| ER | Positive | 1 | |
| | Negative | 2.8 (0.7, 10.7) | 0.1380 |
| Number of LN invasions | 0 | | |
| | 1-3 | 7.2 (1.8, 28.5) | 0.0051 |
| | ≥4 | 5.2 (1.0, 28.0) | 0.0574 |

| With the 18-gene classifier analysis | | | |
|---|---|---|---|
| ER | Positive | 1 | |
| | Negative | 1.6 (0.4, 5.7) | 0.4911 |
| Number of LN invasions | 0 | | |
| | 1-3 (+) | 1.6 (0.4, 6.0) | 0.4952 |
| | ≥4 (+) | 1.3 (0.2, 7.6) | 0.7921 |
| 18-gene score | ≥31 | 67.8 (8.3, 552.5) | <0.0001 |
| | <31 | 1 | |

### Example 4

### Evaluating LRR rates using the (18 gene panel) classifier for BCS patients

Eighty-seven (87) BCS patients were analyzed using microarray information; 94% of these patients (82/87) had been given post-operative radiotherapy (see Table 1). The clinical characteristics of these patients were slightly different from those of T1 mastectomy patients who have received adjuvant chemotherapy.

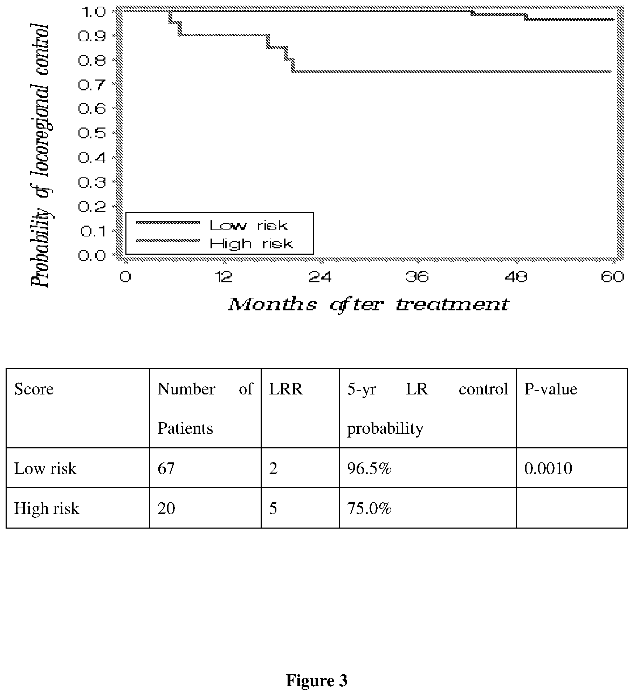

Univariate and multivariate analyses confirmed that the (18 gene panel) classifier could verify whether a BCS patient having accepted post-operative adjuvant chemotherapy was at high risk of LRR. The (18 gene panel) classifier improved the performance of prognosis risk assessment for patients having received no adjuvant chemotherapy. Multivariate analysis showed that an 18-gene score that was ≥31 and prominent LV invasions were independent risk factors of LRR rates. BCS patients receiving no adjuvant chemotherapy had a risk of 40% LRR rate, while patients verified by the (18 gene panel) classifier as in a low risk group had only a 3% LRR rate (see Table 7 and Figure 3). If verified by the (18 gene panel) classifier as in a low risk group, those patients receiving no adjuvant chemotherapy would have no LRR.

**Table 7: Factors associating BCS patients with LRR rates**

| Risk factor | Number of Patients | Number of LRRs | LRR rate | Univariate analysis | Multivariate analysis |
|---|---|---|---|---|---|
| | | | | Hazard ratio (95% CI) | Hazard ratio (95% CI) |
| Age | | | | | |
| <40 | 19 | 2 | 10.5% | 1.4 (0.3, 7.1) | |
| ≥40 | 68 | 5 | 7.4% | | |

| T stage | | | | | |
|---|---|---|---|---|---|
| T1 | 54 | 2 | 3.7% | | |
| T2 | 33 | 5 | 15.2% | 4.3 (0.8, 22.2) | |

| Number of LN invasions | | | | | |
|---|---|---|---|---|---|
| 0 | 56 | 4 | 7.1% | | |
| 1-3 | 23 | 2 | 8.7% | 1.2 (0.2, 6.6) | |
| ≥4 | 8 | 1 | 12.5% | 2.1 (0.2, 19.2) | |

| ER status | | | | | |
|---|---|---|---|---|---|
| Negative | 25 | 3 | 12.0% | 2.0 (0.5, 9.1) | |
| Positive | 62 | 4 | 6.5% | | |
| PR status | | | | | |
| Negative | 32 | 3 | 9.4% | 1.3 (0.3, 5.9) | |
| Positive | 55 | 4 | 7.3% | | |

| HER2 | | | | | |
|---|---|---|---|---|---|
| Negative | 62 | 5 | 8.1% | | |
| Positive | 25 | 2 | 8.0% | 1.2 (0.2, 6.2) | |

| LV invasion | | | | | |
|---|---|---|---|---|---|
| Absent/local | 73 | 4 | 5.5% | | |
| Prominent | 14 | 3 | 21.4% | 4.3 (1.0, 19.3) | 5.0 (1.1, 22.7) |

| Nuclear grade | | | | | |
|---|---|---|---|---|---|
| Grade 1-2 | 42 | 3 | 7.1% | | |
| Grade 3 | 44 | 4 | 9.1% | 1.4 (0.3, 6.3) | |

| classifier (18 gene panel) | | | | | |
|---|---|---|---|---|---|
| Low risk | 67 | 2 | 3.0% | | |
| High risk | 20 | 5 | 25.0% | 9.5 (1.8, 49.2) | 10.4 (2.0, 54.0) |

| Adjuvant Radiotherapy | | | | | |
|---|---|---|---|---|---|
| No | 5 | 2 | 40% | 10.3 (2.0, 53.9) | |
| Yes | 82 | 5 | 6.1% | | |

| Adjuvant Chemotherapy | | | | | |
|---|---|---|---|---|---|
| No | 11 | 0 | 0.0% | | |
| Yes | 76 | 7 | 9.2% | | |

| Adjuvant Hormonal Therapy | | | | | |
|---|---|---|---|---|---|
| No | 26 | 4 | 15.4% | 3.5 (0.8, 15.7) | |
| Yes | 61 | 3 | 4.9% | | |

In conclusion, the (18 gene panel) classifier used in the present invention may verify mastectomy and BCS patients who are at high risk of LRR. In the present invention, the (18 gene panel) classifier may verify whether mastectomy and BCS patients who are at high risk require intervention of radiotherapy.

### Example 5

### Predicting the likelihood of distant metastasis in different subtypes, stages or treatment modalities

### Materials and Methods

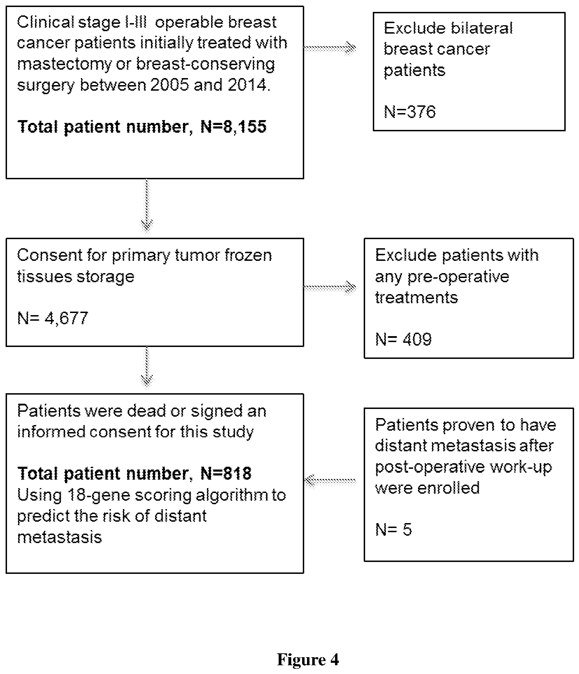

A total of 818 breast cancer patients with operable breast cancer who underwent primary surgery with microarray-based gene expression profile over primary tumor tissues were selected for the study. The patients were clinically stage I-III breast cancer patients who underwent primary surgery from 2005 to 2012 in the Koo Foundation Sun Yat-Sen Cancer Center. The flow of patient selection is shown in Figure 4. Eligible patients in the study should meet the following inclusion criteria: (1) women with invasive carcinoma of breast cancer; (2) clinical stages I-III before treatment; (3) treated with modified mastectomy or total mastectomy and sentinel lymph node biopsy; (4) frozen fresh tissues available; and (5) signed informed consent.

### Microarray study with Affymetrix U133 plus 2.0 arrays

A total number of 818 frozen tissue samples came from surgical specimens of the primary tumors taken from patients prior to any systemic treatment between 2005 and 2014. Hybridization targets were prepared from total RNA and hybridized to U133 plus 2.0 arrays according to the Affymetrix protocol.

### 18 genes and scoring algorithm

The 18-gene panel or classifier includes the genes BLM, TCF3, PIM1, RCHY1, PTI1, DDX39, BUB1B, STIL, TPX2, CCNB1, MMP15, CCR1, NFATC2IP, TRPV6, OBSL1, C16ORF7, DTX2 (Notch) and ENSA. The 18-gene score is calculated based on odds ratio of multivariate analysis. The score of each gene is re-scaled according to the odds ratio to a score between 1 and 11; each gene counts as 1 point when the odds ratio is <1, and so the total score = 56.

The scoring algorithm is as follows: 18-gene score = 4×TRPV6 + 3×DDX39 + 8×BUBlB + CCR1 + STIL + 3×BLM + 11×C16ORF7 + 4×PIM1 + TPX2 + 2×PTI1 + 2×TCF3 + CCNB1 + DTX2 + 2×ENSA + 5×RCHY1 + 4×NFATC2IP + OBSL1 + 2×MMP15; total score =56.

Patients with 18-gene scores of <21 is defined as low risk of distant metastasis, scores of 21 to 43 as intermediate risk of distant metastasis, and scores of ≥44 is defined as high risk of distant metastasis.

### Statistical methods

Cox proportional hazards regression models were used to assess the prognostic significance of the following risk factors: age at diagnosis, primary tumor size, the number of involved axillary lymph nodes, histological grade, nuclear grade, lymphovascular invasion, ER/PR status, HER2 overexpression and 18-gene score. Duration of locoregional control was calculated from the first day of treatment until the day of the chest wall or regional nodal recurrence or last follow-up. Loco/regional control probability (LRCP) was calculated according to the Kaplan-Meier method. Log-rank test was used to assess the statistical significance of the differences in LRCP, relapse-free probability (RFP), distant metastasis-free probability (DMFP) and overall survival between patient subsets.

### Results

### Patients

The baseline characteristics of all 818 patients can be found in Table 8 below. The median follow-up interval for patients without metastasis was 56.4 (0-159.4). The follow-up interval for patients with metastasis (survival patients only) was 61.4 (18.0-168.2) months. The most common age was between 40-60 years old. Primary tumor was usually greater than 2cm (n=484, 59.2%). N0 disease was the most common (n=392, 47.9%), followed by N1 disease (n=248, 30.3%). Most patients were ER or PR positive (n=537, 65.6%) and HER2 negative (n=512, 62.6%). Prominent lymphovascular invasion (LVI) was present in 24.3% of patients (n=199). Tumor grade III (n=461, 56.4%) was the most common, followed by grade II (n=267, 32.6%).

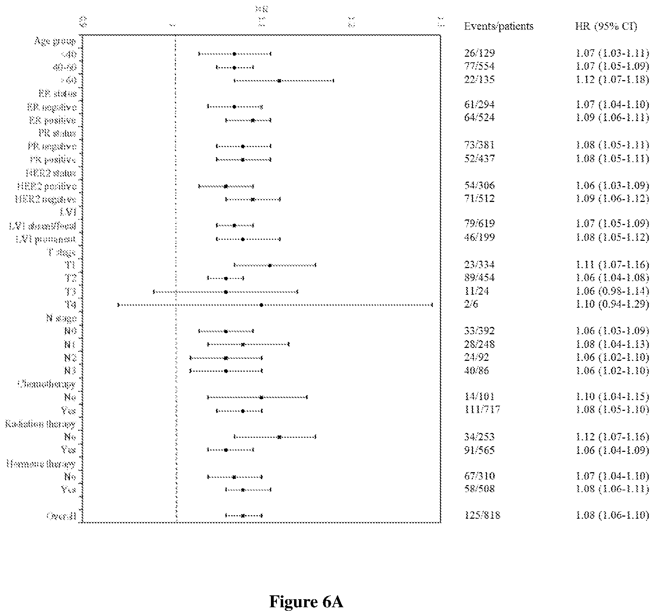

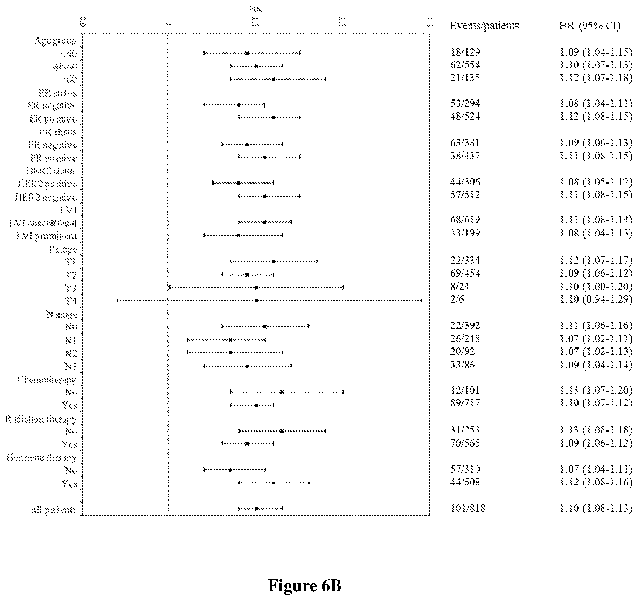

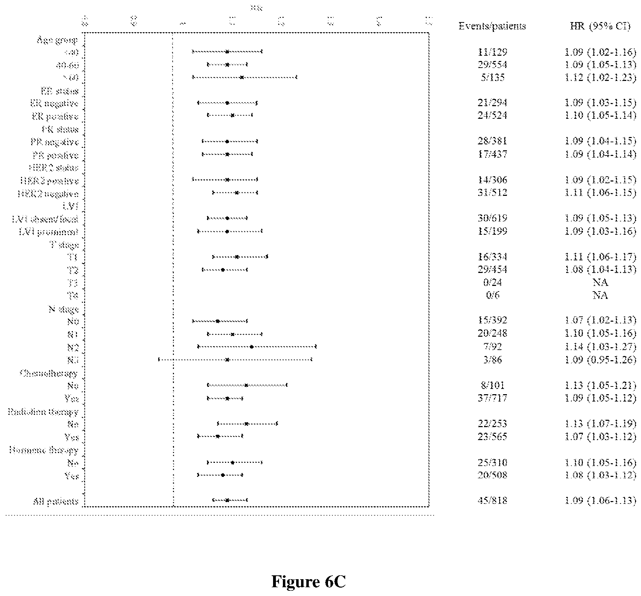

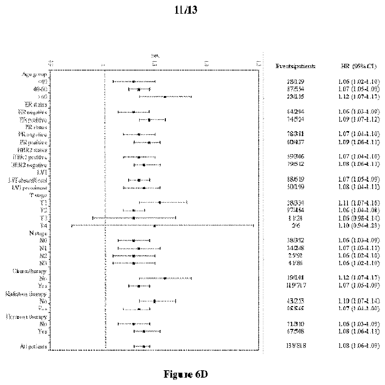

In relation to breast cancer subtypes (see Figure 6), 32.5% (n=266) of patients were luminal-A subtype, 12.6% (n=103) were luminal-B subtype, 20.5% (n=168) were HER2-luminal subtype, 16.9% (n=138) were HER-2 subtype and 17.5% (n=143) were triple-negative subtype.

Adjuvant chemotherapy was administered to 87.7% (n=717) of patients, adjuvant hormonal therapy to 62.1% (n=508) and adjuvant radiotherapy to 69.1% (n=565).

As shown in Table 1 below, the risk factors that are significantly related to distant metastasis include higher tumor stage, more advanced nodal stage, both hormonal receptor negative and prominent LVI.

### 18-gene Classifier

According to the 18-gene scoring algorithm, 21.9% (n=179) of patients were classified to be low risk (scores of <21), 57.5% (n=470) to be intermediate risk (scores of 21-43), and 20.7% (n=169) of patients to be high risk (scores of ≥44). The distant metastasis rates in the low-, intermediate- and high-risk group were 2.2%, 14.3% and 32%, respectively (p<0.0001, see Table 1).

The univariate and multivariate analysis of factors associated with distant metastasis by Cox's Proportional Hazard Regression Models can be found in Table 9 below.

Univariate analysis for distant metastasis by Cox proportional hazards regression model revealed that the hazard ratio was 8.9 (3.3-24.2) for the patients with 18-gene scores of ≥21 (Table 9). In multivariate analysis, the hazard ratio was 5.7 (2.0-15.9). Other risk factors independently related to distant metastasis included T stage (T3 vs. T1, HR 3.8, 95% CI 1.8-8.1) and N stage (N3 vs. N0, HR 5.9, 95% CI 3.3-10.7). No adjuvant treatments (hormonal therapy, radiotherapy and chemotherapy) increased the risk of distant metastasis with the hazard ratios of 2.3 (1.1, 4.9) for hormonal therapy, 2.1 (1.1, 3.9) for chemotherapy, and 1.8 (1.1, 3.0) for radiotherapy, respectively (Table 9).

### Performance of 18-gene classifier in different stage, subtype and subgroup

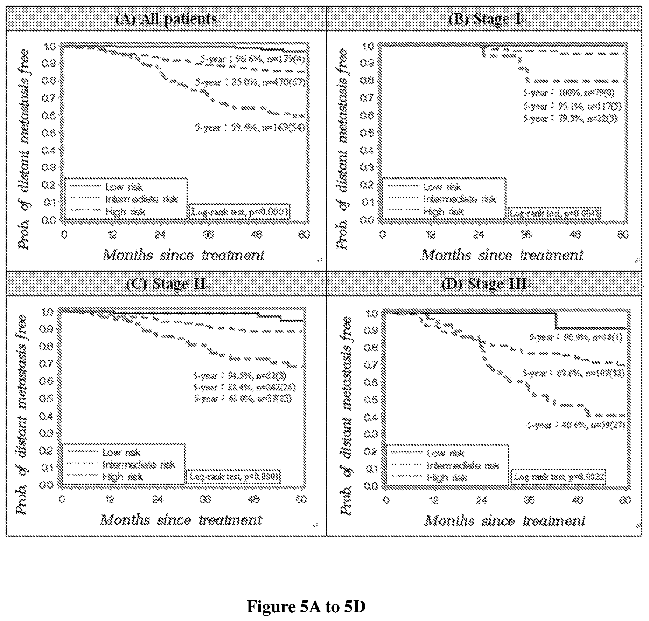

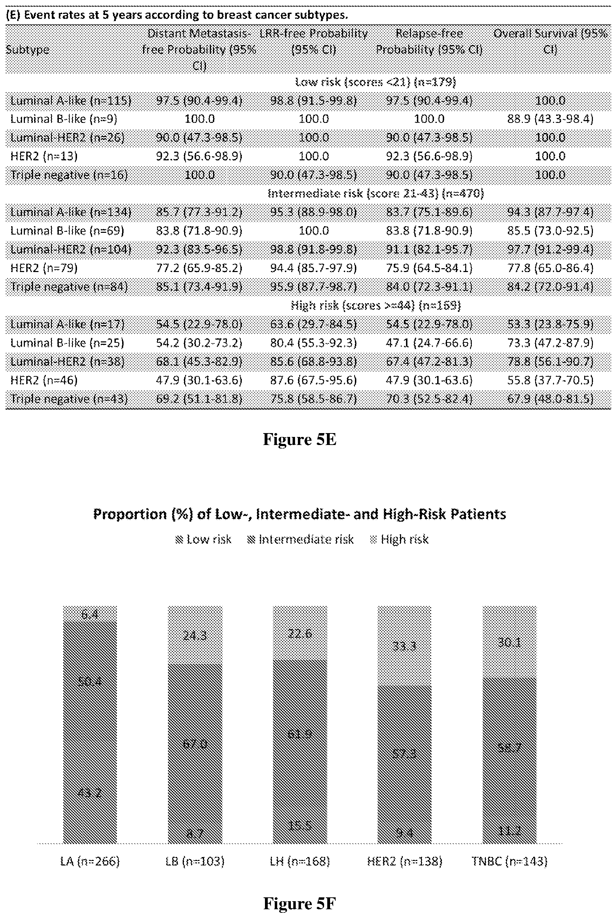

Figure 5A shows the rates of 5-year DMFP of the low-, intermediate-, and high-risk groups were 96.6%, 85.0% and 59.6%, respectively. The percentages of low-risk group analyzed with the 18-gene classifier in stage I, II and III were 36.2% (79/218), 19.9% (82/411) and 9.7% (18/184), respectively (Figure 5). In patients with scores of <21 (the low-risk group), the 5-year rates of DMFP in stage I, II and III were 100%, 94.5% and 90.9%. In the high-risk group (scores of ≥44), the corresponding rates of 5-year DMFP were 79.3%, 68.0% and 40.6%, respectively.

Figure 5E shows the proportions of low-risk patients in luminal-A like, luminal-B like, luminal-HER2, HER2 and triple-negative subtype were 43.2% (115/266), 8.7% (9/103), 15.5% (26/168), 9.4% (13/138) and 11.2% (16/143), respectively. As shown in Figure 5E, the rates of 5-year DMFP of low-risk group in luminal-A like, luminal-B like, luminal-HR2, HER2 and triple-negative subtype were 97.5%, 100%, 90%, 92.3% and 100%. The corresponding rates of 5-year RFP were 98.8%, 100%, 100%, 100% and 90%, respectively. In the high-risk group, the corresponding rates of 5-year DMFP were 54.5%, 54.2%, 68.1%, 47.9% and 69.2%. The mean 18-gene scores in luminal-like subtype were lower (26.8) than those of HER2 subtype (35.6) and triple-negative subtype (36.7).

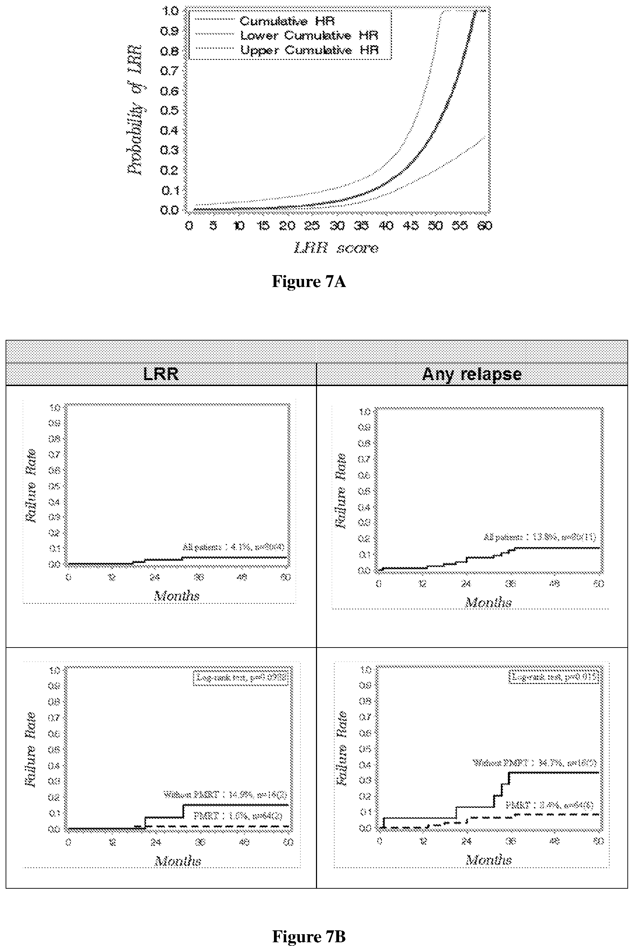

Using the 18-gene score as a continuous variable, Figure 7 demonstrates that the 18-gene classifier is an independent prognostic factor that related to distant recurrence, LRR, any recurrence and overall survival. This observation is across the board in different sets of age groups (<40, 40-60 and >60), stages (except T3 and T4), subtypes (ER, PR, and HER2 status) and treatment modalities (chemotherapy, radiotherapy and hormone therapy). The hazard ratio is around 1.06 to 1.14. The risk of recurrence will increase 1.06 to 1.14 per score increment.

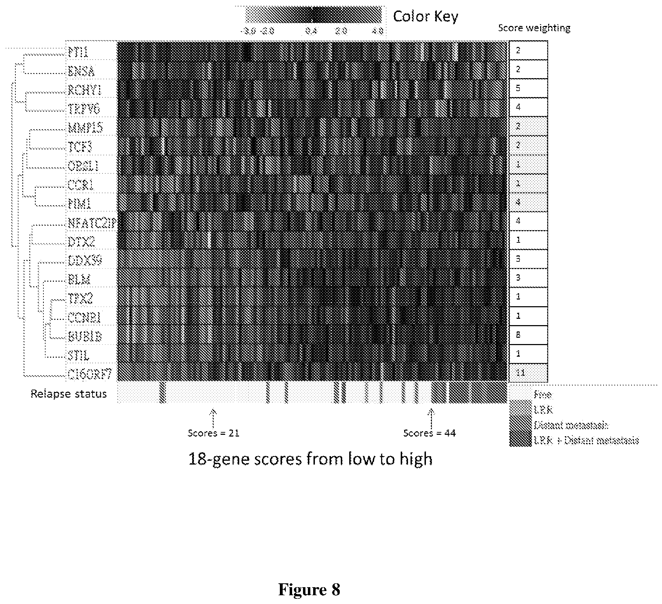

In another embodiment of the present invention, a way of representing gene expression profiles in patients with and without recurrence of LRR and/or distant metastasis is in the form of a dendrogram (see Figure 8). An unsupervised cluster analysis of 18 genes and supervised clustering in 135 patients according to the 18-gene scores revealed distinct gene expression profiles in patients with and without recurrence. Patients with locoregional recurrence (LRR) are in blue ( 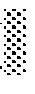 ), both LRR and distant metastasis are in purple ( 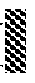 ), distant metastasis patients are in red ( 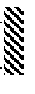 ), and disease free patients are in yellow ( ) as shown at the bottom of the heatmap of Figure 8.

### Discussion

The 18-gene classifier is a multifunctional gene panel that is capable of predicting distant metastasis regardless of cancer subtype, stage, surgical types or adjuvant treatments. The rates of 5-year DMFP in low-, intermediate- and high-risk group were 96.6%, 85% and 59.6%, respectively. Adjusted by other clinical and pathological variables, 18-gene classifier is an independent prognostic factor for distant metastasis with hazard ratio of 5.7 (95%CI, 2.0 - 15.9) (Table 9). Forest plots confirmed that the 18-gene classifier is significantly related to LRR, distant metastasis, any relapse and mortality in different subgroups (Figures 5A-D). Using the 18-gene score as a continuous variable, the hazard ratio for distant metastasis was 1.08 (95% CI, 1.06 to 1.1), hazard ration for the LRR was 1.09 (1.06 to 1.13), hazard ratio for any recurrence was 1.08 (1.06-1.09) and hazard ratio for the mortality was 1.09 (1.06-1.13) per score increment. The 18-gene classifier simultaneously possesses the power to be a prognostic biomarker for both distant metastasis and LRR.

The study group of 818 patients was selected from an initial group of 8,155 patients, which represent a randomly selected breast cancer population in a free-standing cancer center, which treated about one-tenth of breast cancer patients in Taiwan [16, 17]. Unlike other multigene panel trials focused on a specific subtype and stage of breast cancer, the study provides that 18-gene classifier likely is a universal multigene panel with good or bad prognostic value for distant metastasis in general population with breast cancer.

The distant metastatic rate in this study group is higher than an initial group due to those patients with mortality or recurrence will be enrolled in the study first (Figure 4). However, the result of this cohort is compatible with the common consensus that 1) higher T and N stage increase likelihood of distant metastasis; 2) adjuvant treatments decrease likelihood of distant metastasis; 3) triple-negative subtype is more aggressive than luminal-like breast cancer with higher 18-gene scores and recurrence rate.

The 18-gene classifier is capable of revealing overall 22% low-risk breast cancer patients with a 5-year distant metastasis rate less than 4%. There are three unique characteristics of the 18-gene classifier. First, it is the first Asian-based genomic panel validating on large number of general population with breast cancer. Second, it appears to stratify the relapse-risk in non-luminal breast cancer. Third, it can simultaneously predict both the likelihood of LRR and distant metastasis.

During the past 15 years, there is a consensus that early-stage breast cancer is a heterogeneous disease with different molecular subtypes and prognoses [13,18,19]. As discussed in the background section, several multigene panels for early-stage breast cancer have been developed to date [15]. Subsequent multigene panels (Prosigna, EndoPredict, Breast Cancer Index) are claimed to be better than the formers (OncotypeDx, MammaPrint, Genomic Grade Index) for prediction of late distant metastasis [20,21]. These focus mainly on luminal-like (ER positive/HER2 negative) and node-negative breast cancer, which lead to the possibility of omitting chemotherapy in the low-risk group of the subsets.

However, the possibility of stratifying the risk of relapse in non-luminal breast cancer patients remained an unresolved question based on existing panels. For example, the prognostic risk discrimination of the 70-gene and 76-gene panels is good among ER-positive patients, but less than 5% of ER-negative patients are classified as low-risk group [13,14]. Relatively high (>50%) false negative rate for HER-2 subtype by 21-gene panel was also noted [22]. This may be due to first-generation genomic panel relay largely on quantification of proliferation-related genes to determine the prognosis of ER-positive disease [23]. As a result, these research confines the clinical utility of multigene panels to other breast cancer subtypes. Several studies have shown that the gene expression profiles related to immune response and stromal invasion have prognostic value for ER-negative and high proliferative ER-positive breast cancer [24-27]. However, those classified by second-generation genomic panel as low-risk group remained a 5-year relapse rate up to 20%.

The 18 genes of the 18-gene classifier are associated with proliferation, oncogenic process, invasion, inflammation, cell-cell interaction, apoptosis and metabolism. In this study, the 18-gene classifier identified 12.7% HER2 (+) and 11.2% triple negative breast cancer (TNBC) patients as the low-risk group with 5-year distant metastasis rate of 8% and 0%, respectively. This extraordinary result provides the possibility of stratifying the relapsed-risk of non-luminal breast cancer. If so, reducing or withholding the dosage of chemotherapy in this disease subset may be possible. Other well-noted information is that in the 18-gene classifier's high-risk group, the 5-year DMFP is only 40-60% regardless of treatment, dose-dense chemotherapy or novel clinical trial may be considered in these patients.

Moreover, racial disparities of breast cancer are another issue [28]. Asian Women have better survival rate than that of Non-Hispanic Caucasian and African American in some areas, which may lead to overestimate the risk of Asian population with Western genomic panel [29].

The present 18-gene classifier is also able to identify a group of high-risk patients who may benefit from dose-dense chemotherapy and high dose of radiotherapy. The 5-year distant metastasis rate in the low risk group is only 2.2% (4/179). From previous study, adjuvant chemotherapy may reduce the risk of recurrence by 30-50%. Assuming that the greatest odds is taken into consideration, say 50%, the initial distant metastasis rate without chemotherapy in the low-risk group may be around 4.4%. Increasing by less than 3%, there is left the other 95% patients under the toxic effect of chemotherapy in the low-risk group.

Taken together, the 18-gene classifier is a universal panel that is capable of predicting LRR and distant metastasis simultaneously.

**Table 8: Baseline Characteristics of all 818 patients**

| **Variables** | **Absence of metastasis (N=693)** | **Presence of metastasis (N=125)** | **P value** |
|---|---|---|---|
| Median follow-up (Months) | 56.4 (0-159.4) | 61.4 (18.0-168.2) | 0.3391* |

| **Age** | | | 0.1928 |
|---|---|---|---|
| <40 | 103 (79.8%) | 26 (20.2%) | |
| 40-60 | 477 (86.1%) | 77 (13.9%) | |
| >60 | 113 (83.7%) | 22 (16.3%) | |

| **T stage** | | | **<0.0001** |
|---|---|---|---|
| T1 | 311 (93.1%) | 23 (6.9%) | |
| T2 | 365 (80.4%) | 89 (19.6%) | |
| T3 | 13 (54.2%) | 11 (45.8%) | |
| T4 | 4 (66.7%) | 2 (33.3%) | |

| **N stage** | | | **<0.0001** |
|---|---|---|---|
| N0 | 359 (91.6%) | 33 (8.4%) | |
| N1 | 220 (88.7%) | 28 (11.3%) | |
| N2 | 68 (73.9%) | 24 (26.1%) | |
| N3 | 46 (53.5%) | 40 (46.5%) | |

| **ER and PR status** | | | **0.0002** |
|---|---|---|---|
| Both Negative | 220 (78.3%) | 61 (21.7%) | |
| ER or PR positive | 473 (88.1%) | 64 (11.9%) | |

| **HER2 overexpression**** | | | **0.146** |
|---|---|---|---|
| Negative | 441 (86.1%) | 71 (13.9%) | |
| Positive | 252 (82.4%) | 54 (17.7%) | |

| **Lymphovascular invasion** | | | **0.0004** |
|---|---|---|---|
| Absent/focal | 540 (87.2%) | 79 (12.8%) | |
| Prominent | 153 (76.9%) | 46 (23.1%) | |

| **Tumor grade** | | | **0.0009** |
|---|---|---|---|
| Grade I | 86 (95.6%) | 4 (4.4%) | |
| Grade II | 233 (87.3%) | 34 (12.7%) | |
| Grade III | 374 (81.1%) | 87 (18.9%) | |

| **Adjuvant chemotherapy** | | | **0.6719** |
|---|---|---|---|
| No | 87 (86.1%) | 14 (13.9%) | |
| Yes | 606 (84.5%) | 111 (15.5%) | |

| **Adjuvant H/T** | | | **<0.0001** |
|---|---|---|---|
| No | 243 (78.4%) | 67 (21.6%) | |
| Yes | 450 (88.6%) | 58 (11.4%) | |

| **Adjuvant radiotherapy** | | | **0.3271** |
|---|---|---|---|
| No | 219 (86.6%) | 34 (13.4%) | |
| Yes | 474 (83.9%) | 91 (16.1%) | |

| **18-gene score** | | | **<0.0001** |
|---|---|---|---|
| Low risk (<21) | 175 (97.8%) | 4 (2.2%) | |
| Intermediate (21-43) | 403 (85.7%) | 67 (14.3%) | |
| High risk (≥44) | 115 (68.1%) | 54 (32.0%) | |

| | | | |
|---|---|---|---|
| *Use two-sample t-test to test the mean follow-up time of the two groups. **6 patients' HER2 status was reclassified according to molecular expression level. | | | |

**Table 9: Univariate and Multivariate analysis of factors associated with distant metastasis by Cox's Proportional Hazard Regression Models (N=818)**

| **Variable** | | **Hazard ratio (95% CI) (Crude)** | **P value** | **Hazard ratio (95% CI) (Adjusted)** | **P value** |
|---|---|---|---|---|---|
| **ER status** | Negative | **1.7 (1.2,2.4)** | 0.0031 | **0.7 (0.3,1.5)** | 0.3883 |
| | Positive | 1 | | 1 | |
| **PR status** | Negative | **1.6 (1.1, 2.3)** | 0.01 | **0.8 (0.4, 1.4)** | 0.4454 |
| | Positive | 1 | | 1 | |
| **HER2 status** | Negative | 1 | 0.0395 | 1 | 0.9854 |
| | Positive | **1.5 (1.0, 2.1)** | | **1.0 (0.7, 1.5)** | |
| **T stage** | T2 vs. T1 | **2.8 (1.7, 4.4)** | <.0001 | **1.9 (1.1, 3.0)** | 0.0124 |
| | T3 vs. T1 | **6.8 (3.3, 14.1)** | <.0001 | **3.8 (1.8, 8.1)** | 0.0007 |
| | T4 vs. T1 | **5.4 (1.3, 22.8)** | 0.0228 | 1.9 (0.4, 8.3) | 0.4043 |
| **N stage** | N1 vs. N0 | 1.3 (0.8, 2.2) | 0.2555 | 1.5 (0.8, 2.6) | 0.1793 |
| | N2 vs. N0 | **3.5 (2.0, 5.8)** | <.0001 | **3.8 (2.0, 7.4)** | <.0001 |
| | N3 vs. N0 | **6.5 (4.1, 10.3)** | <.0001 | **5.9 (3.3,10.7)** | <.0001 |
| **Tumor grade** | I-II | 1 | 0.0003 | 1 | 0.5822 |
| | III | **2.0 (1.4, 3.0)** | | 1.1 (0.7, 1.8) | |
| **LVI** | No/focal | 1 | 0.0008 | 1 | 0.8226 |
| | Prominent | **1.9 (1.3, 2.7)** | | 1.0 (0.7, 1.6) | |
| **Hormone therapy** | No | **2.0 (1.4, 2.9)** | <.0001 | **2.3 (1.1, 4.9)** | 0.0262 |
| | Yes | 1 | | 1 | |
| **Radiotherapy** | No | 0.8 (0.5, 1.2) | 0.2417 | **1.8 (1.1, 3.0)** | 0.0165 |
| | Yes | 1 | | 1 | |
| **Chemotherapy** | No | 1.0 (0.6, 1.7) | 0.951 | **2.1 (1.1, 3.9)** | 0.0203 |
| | Yes | 1 | | 1 | |
| **18-gene score** | <21 | 1 | <.0001 | 1 | 0.0009 |
| | ≥21 | **8.9 (3.3, 24.2)** | | **5.7 (2.0, 15.9)** | |

### List 1: Gene coding sequences for each of the 18 genes of the 18-gene classifier

1. **TRPV6:** transient receptor potential cation channel subfamily V member 6 [Homo sapiens (human)] NCBI Reference Sequence: NM_014274.3 (SEQ ID NO: 1)
2. **DDX39:** DEAD-box helicase 39A [Homo sapiens (human)] (SEQ ID NO: 2)
3. **BUB1B:** BUB1 mitotic checkpoint serine/threonine kinase B [Homo sapiens (human)] (SEQ ID NO: 3)
4. **CCR1:** C-C motif chemokine receptor 1 [Homo sapiens (human)] NCBI Reference Sequence: NM_001295.2 (SEQ ID NO: 4)
5. **STIL:** SCL/TAL1 interrupting locus [Homo sapiens (human)] NCBI Reference Sequence: NM_003035.2 (SEQ ID NO: 5)
6. **BLM:** Bloom syndrome RecQ like helicase [Homo sapiens (human)] NCBI Reference Sequence: NM_000057.3 (SEQ ID NO: 6)
7. **C16ORF7(previous name):** VPS9 domain containing 1 [Homo sapiens (human)] NCBI Reference Sequence: NM_004913.2 (SEQ ID NO: 7)
8. **TPX2:** TPX2, microtubule nucleation factor [Homo sapiens (human)] NCBI Reference Sequence: NM_012112.4 (SEQ ID NO: 8)
9. **PTI1 (also known as EEF1A1**): eukaryotic translation elongation factor 1 alpha 1 [Homo sapiens (human)] NCBI Reference Sequence: NM_001402.5 (SEQ ID NO: 9)
10. **TCF3:** transcription factor 3 [Homo sapiens (human)] NCBI Reference Sequence: NM_003200.3 (SEQ ID NO:10)
11. **CCNB1:** cyclin B1 [Homo sapiens (human)] NCBI Reference Sequence: NM_031966.3 (SEQ ID NO: 11)
12. **DTX2:** deltex 2, E3 ubiquitin ligase [Homo sapiens (human)] NCBI Reference Sequence: NM_020892.2 (SEQ ID NO: 12)
13. **PIM1:** Pim-1 proto-oncogene, serine/threonine kinase [Homo sapiens (human)] NCBI Reference Sequence: NM_002648.3 (SEQ ID NO: 13)
14. **ENSA:** endosulfine alpha [Homo sapiens (human)] NCBI Reference Sequence: NM_207042.1 (SEQ ID NO: 14)
15. **RCHY1:** ring finger and CHY zinc finger domain containing 1 [Homo sapiens (human)] NCBI Reference Sequence: NM_015436.3 (SEQ ID NO: 15)
16. **NFATC2IP:** nuclear factor of activated T-cells 2 interacting protein [Homo sapiens (human)] NCBI Reference Sequence: NM_032815.3 (SEQ ID NO: 16)
17. **OBSL1:** obscurin-like 1 [Homo sapiens (human)] NCBI Reference Sequence: NM_015311.2 (SEQ ID NO: 17)
18. **MMP15:** matrix metallopeptidase 15 [Homo sapiens (human)] NCBI Reference Sequence: NM_002428.3 (SEQ ID NO: 18)

### Benefits of the Present Invention

PMRT is given to a patient once per day and 5 times per week. It usually takes 4 to 6 weeks to complete a course of treatment. Such a treatment brings about a heavy burden on not just the patients, but also their families and the society, which include commuting for treatment daily, absence from workplace, rearrangement of manpower due to employees on leave, extra-financial expense, etc. The prognostic test and kit of the present invention can provide predictions with higher accuracy and precision which will impact adjuvant treatment decisions and reduce or prevent excessive treatment or overtreatment by identifying individuals with low risk of recurrence and/or distant metastasis for whom adjuvant therapies such as PMRT, chemotherapy and hormonal therapy can be avoided. The reduction or prevention of excessive treatment or overtreatment would advantageously reduce or prevent the unnecessary side effects associated with excessive treatment or overtreatment. Consequently, the associated burdens on the patients, their families and the society may be relieved.

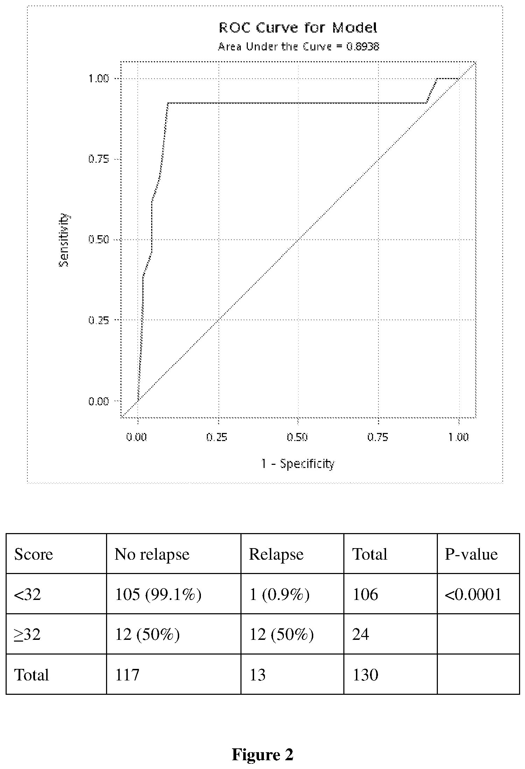

Although the (18 gene panel) classifier used in the present invention shares some basic concepts with the 34-gene set prediction model shown in Figure 2, the present invention provides an evaluation kit with high accuracy, sensitivity and specificity that can accurately predict and verify. Clinically, the (18 gene panel) classifier provides an effective auxiliary tool for evaluation to predict potential LRR risk for patients.

Furthermore, the 18-gene classifier is also able to predict the likelihood of distant metastasis in breast cancer patients. Based on the scoring algorithm, patients with 18-gene scores of <21 is defined as low risk of distant metastasis, scores of 21 to 43 as intermediate risk of distant metastasis, and scores of ≥44 is defined as high risk of distant metastasis. Advantageously, the primary outcome is 5-year probability of freedom from distant metastasis (DMFP).

With median follow-up interval of 56.7 (0 to 168.2) months, the 5-year rates of DMFP for patients classified as low risk (n=179/818, 21.9%), intermediate risk (n=470/818, 57.5%) and high risk (n=169/818, 20.7%) were 96.6%, 85% and 59.6%, respectively. The 5-year rate of DMFP of the low-risk group in stage I (n=79/218, 36.2%) was 100%, the rate in stage II (n=82/411, 19.9%) was 94.5%, and the rate in stage III (n=18/184, 9.7%) was 90.9%. Multivariate analysis revealed that the 18-gene classifier is an independent prognostic factor that affects distant metastasis regardless of age, cancer subtypes, tumor grade, nodal status or adjuvant treatments with adjusted hazard ratio (HR) of 5.7 (95% CI, 2.0 to 15.9; p=0.0009) for scores of ≥21.

It is typically accepted in the industry that for any kind of risk prediction, such a prediction is generally made based on a 5% risk (or confidence of 95%). In the present invention, when predicting the likelihood of LRR and/or distant metastasis in a subject with breast cancer following mastectomy and/or breast conserving surgery, the suggested cut-off value for risk stratification of LRR and distant metastasis is based on a 5% risk (or confidence of 95%). However, it would be appreciated that if there is a preference or desire to be more conservative or less conservative, the cut-off value would change accordingly.

For example, in one or more of the embodiments described above, the optimal cut-off value was score 31 for classifying whether a subject falls under a low risk or high risk group of LRR based on the scoring algorithm, in which scores less than 31 is defined as low risk of LRR and scores ≥31 is defined as high risk of LRR.

In another example, in one or more of the embodiments described above, based on the scoring algorithm, subjects with scores of <21 is defined as low risk of distant metastasis, scores of 21 to 43 as intermediate risk of distant metastasis, and scores of ≥44 is defined as high risk of distant metastasis.

The reference score 31 (or first pre-determined reference) mentioned above can also be used to classify whether a subject falls under a low risk or high risk group of distant metastasis. However, should such a score be used for risk classification of distant metastasis, the overall accuracy and sensitivity and specificity would be affected (i.e. increased or decreased).

Similarly, the scores of <21 (or second pre-determined reference), 21 to 43, and ≥44 (or third pre-determined reference) mentioned above can also be used to classify whether a subject falls under a low risk, intermediate risk or high risk group of LRR. However, should such scores be used for risk classification of LRR, the overall accuracy and sensitivity and specificity would be affected (i.e. increased or decreased).

Taken together, although there are described preferred or optimal scores for risk classification for LRR and distant metastasis, it is possible to vary the optimal reference scores depending on the preferred or desired degree of conservativeness or risk appetite.

Advantageously, the 18-gene classifier is a universal prognostic biomarker for breast cancer to predict or estimate the risk of LRR and/or distant metastasis in breast cancer patients.

Current medical practice is to give treatment by static average. While some treatments indeed provide benefits, others do not. This causes a big waste of medical resources. By contrast, the present invention advantageously provides a precise and effective evaluation method, system and kit that facilitate precise and effective use of medical and insurance resources.

### Other Embodiments

A kit according to an aspect of the present disclosure may contain antibodies, aptamers, amplification systems, detection reagents (chromogen, fluorophore, etc.), dilution buffers, washing solutions, counter stains or any combination thereof. Kit components may be packaged for either manual or partially or wholly automated practice of the foregoing methods. In other embodiments involving kits, this invention contemplates a kit including compositions of the present invention, and optionally instructions for their use. Such kits may have a variety of uses, including, for example, stratifying patient populations, diagnosis, prognosis, guiding therapeutic treatment decisions, and other applications.

All of the features disclosed in this specification may be combined in any combination. Each feature disclosed herein may be replaced by an alternative feature serving the same, equivalent, or similar purposes. Thus, unless expressly stated otherwise, each feature disclosed is only an example of a generic series of equivalent or similar features.

In view of the foregoing description, it is to be understood that the above embodiments have been provided only by way of exemplification of the present invention. Thus, other embodiments are also within the claims.

### References

1. Taghian A, Jeong JH, Mamounas E, et al. Patterns of locoregional failure in patients with operable breast cancer treated by mastectomy and adjuvant chemotherapy with or without tamoxifen and without radiotherapy: results from five National Surgical Adjuvant Breast and Bowel Project randomized clinical trials. J Clin Oncol 2004;22:4247-54.
2. Cheng SH, Horng CF, Clarke JL, et al. Prognostic index score and clinical prediction model of local regional recurrence after mastectomy in breast cancer patients. Int J Radiat Oncol Biol Phys 2006;64:1401-9.
3. Zellars RC, Hilsenbeck SG, Clark GM, et al. Prognostic value of p53 for local failure in mastectomy-treated breast cancer patients. J Clin Oncol 2000;18:1906-13.
4. van der Hage JA, van den Broek LJ, Legrand C, et al. Overexpression of P70 S6 kinase protein is associated with increased risk of locoregional recurrence in node-negative premenopausal early breast cancer patients. Br J Cancer 2004;90:1543-50.
5. Recht A, Edge SB, Solin LJ, et al. Postmastectomy radiotherapy: clinical practice guidelines of the American Society of Clinical Oncology. J Clin Oncol 2001;19:1539-69.
6. Overgaard M, Hansen PS, Overgaard J, et al. Postoperative radiotherapy in high-risk premenopausal women with breast cancer who receive adjuvant chemotherapy. Danish Breast Cancer Cooperative Group 82b Trial. N Engl J Med 1997;337:949-55.
7. Overgaard M, Jensen MB, Overgaard J, et al. Postoperative radiotherapy in high-risk postmenopausal breast-cancer patients given adjuvant tamoxifen: Danish Breast Cancer Cooperative Group DBCG 82c randomised trial. Lancet 1999;353:1641-8.
8. Ragaz J, Jackson SM, Le N, et al. Adjuvant radiotherapy and chemotherapy in node-positive premenopausal women with breast cancer. N Engl J Med 1997;337:956-62.
9. EBCTCG, McGale P, Taylor C, et al. Effect of radiotherapy after mastectomy and axillary surgery on 10-year recurrence and 20-year breast cancer mortality: meta-analysis of individual patient data for 8135 women in 22 randomised trials. Lancet 2014;383:2127-35.
10. Paik S, Shak S, Tang G, et al. A multigene assay to predict recurrence of tamoxifen-treated, node-negative breast cancer. N Engl J Med 2004;351:2817-26.
11. Cheng SH, Horng CF, West M, et al. Genomic prediction of locoregional recurrence after mastectomy in breast cancer. J Clin Oncol 2006;24:4594-602.
12. Solin LJ, Gray R, Baehner FL, et al. A multigene expression assay to predict local recurrence risk for ductal carcinoma in situ of the breast. J Natl Cancer Inst 2013;105:701-10.
13. van't Veer LJ, Dai H, van de Vijver MJ, et al, Gene expression profiling predicts clinical outcome of breast cancer, Nature 2002, 415:530-6.
14. Wang Y, Klijn JG, Zhang Y, et al, Gene-expression profiles to predict distant metastasis of lymph-node-negative primary breast cancer, Lancet 2005, 365:671-9.
15. Harris LN, Ismaila N, McShane LM, et al, Use of Biomarkers to Guide Decisions on Adjuvant Systemic Therapy for Women With Early-Stage Invasive Breast Cancer: American Society of Clinical Oncology Clinical Practice Guideline, Journal of Clinical Oncology 2016.
16. CANCER REGISTRY ANNUAL REPORT, 2012 TAIWAN. In: TAIWAN HPAMOHAW, ed. Taipei2015:p 68-9.
17. Porter ME, BARON JF, WANG CJ. Koo Foundation Sun Yat-Sen Cancer Center: Breast Cancer Care in Taiwan. Harvard Business School Review 2010.
18. Sorlie T, Perou CM, Tibshirani R, et al. Gene expression patterns of breast carcinomas distinguish tumor subclasses with clinical implications. Proc Natl Acad Sci U S A 2001;98:10869-74.
19. Perou CM, Sorlie T, Eisen MB, et al. Molecular portraits of human breast tumours. Nature 2000;406:747-52.
20. Mook S, Schmidt MK, Viale G, et al. The 70-gene prognosis-signature predicts disease outcome in breast cancer patients with 1-3 positive lymph nodes in an independent validation study. Breast Cancer Res Treat 2009;116:295-302.
21. Dubsky P, Brase JC, Jakesz R, et al. The EndoPredict score provides prognostic information on late distant metastases in ER+/HER2- breast cancer patients. Br J Cancer 2013;109:2959-64.
22. Dabbs DJ, Klein ME, Mohsin SK, Tubbs RR, Shuai Y, Bhargava R. High false-negative rate of HER2 quantitative reverse transcription polymerase chain reaction of the Oncotype DX test: an independent quality assurance study. J Clin Oncol 2011;29:4279-85.
23. Reis-Filho JS, Pusztai L. Gene expression profiling in breast cancer: classification, prognostication, and prediction. Lancet 2011;378:1812-23.
24. Reyal F, van Vliet MH, Armstrong NJ, et al. A comprehensive analysis of prognostic signatures reveals the high predictive capacity of the proliferation, immune response and RNA splicing modules in breast cancer. Breast Cancer Res 2008;10:R93.
25. Teschendorff AE, Caldas C. A robust classifier of high predictive value to identify good prognosis patients in ER-negative breast cancer. Breast Cancer Res 2008;10:R73.
26. Bianchini G, Qi Y, Alvarez RH, et al. Molecular anatomy of breast cancer stroma and its prognostic value in estrogen receptor-positive and -negative cancers. J Clin Oncol 2010;28:4316-23.
27. Finak G, Bertos N, Pepin F, et al. Stromal gene expression predicts clinical outcome in breast cancer. Nat Med 2008;14:518-27.
28. Albain KS, Unger JM, Crowley JJ, Coltman CA, Hershman DL. Racial Disparities in Cancer Survival Among Randomized Clinical Trials Patients of the Southwest Oncology Group. Journal of the National Cancer Institute 2009.
29. Carey LA, Perou CM, Livasy CA, et al. Race, breast cancer subtypes, and survival in the Carolina Breast Cancer Study. JAMA 2006;295:2492-502.

## Claims

1. A method for predicting the likelihood of locoregional recurrence (LRR) and/or distant metastasis in a subject with breast cancer following mastectomy and/or breast conserving surgery, comprising:
i. measuring the expression level of at least one gene or a plurality of genes in a sample isolated from the subject, wherein the at least one gene or plurality of genes is selected from a group consisting of: TRPV6, DDX39, BUB1B, CCR1, STIL, BLM, C16ORF7, TPX2, PTI1, TCF3, CCNB1, DTX2, PIM1, ENSA, RCHY1, NFATC2IP, OBSL1, MMP15;
ii. deriving a score based on the measured expression level of the at least one gene or the plurality of genes, wherein deriving the score is performed using a predictive classification model, the predictive classification model comprising at least one scoring algorithm.; and
iii. classifying the subject based on the derived score into one of the following groups:
(i) a low risk group of LRR and/or distant metastasis when the derived score is less than a first pre-determined reference; a high risk group of LRR and/or distant metastasis when the derived score is equal to or more than the first pre-determined reference;
(ii) a low risk group of distant metastasis and/or LRR when the derived score is less than a second pre-determined reference;
(iii) a high risk group of distant metastasis and/or LRR when the derived score is equal to or more than a third pre-determined reference; and/or
(iv) an intermediate risk group of distant metastasis and/or LRR when the derived score is between the second pre-determined reference (inclusive) and the third pre-determined reference;
wherein the at least one scoring algorithm is selected from a group consisting of:
i. Score = TRPV6 + DDX39 + BUB1B + CCR1 + STIL + BLM + C16ORF7 + TPX2 + PTI1 + TCF3 + CCNB1 + DTX2 + PIM1 + ENSA + RCHY1 + NFATC2IP + OBSL1 + MMP15; wherein each gene counts as one point when the hazard ratio is <1, and the total score = 18;
ii. Score = TRPV6 + DDX39 + BUB1B + CCR1 + STIL + BLM + 2×C16ORF7 + TPX2 + PTI1 + TCF3 + CCNB1 + DTX2 + PIM1 + ENSA + RCHY1 + 2×NFATC2IP + OBSL1 + 2×MMP15; wherein when the hazard ratio is <1, each gene counts as one point if the genes are examined by univariate analysis, and each gene counts as two points if the genes are examined by multivariate analysis, and so the total score = 21;
iii. Score = 3×TRPV6 + 5×DDX39 + 18×BUB1B + 4×CCR1 + 4×STIL + 7×BLM + 7×C16ORF7 + 4×PMI1 + 9×TPX2 + 8×PTI1 + 3×TCF3 + 7×CCNB1 + 2×DTX2 + 2×ENSA + 5×RCHY1 + 6×NFATC2IP + 2×OBSL1 + 6×MMP15; wherein the genes are examined by univariate analysis and the score of each gene is re-scaled according to its weighting, and so the total score = 102;
iv. Score = 2×TRPV6 + 2×DDX39 + 2×BUB1B + CCR1 + STIL + 2×BLM + 5×C16ORF7 + 3×PIM1 + 3×TPX2 + 5×PTI1 + TCF3 + CCNB1 + DTX2 + 2×ENSA + 3×RCHY1 + 4×NFATC2IP + OBSL1 + MMP15; wherein the genes are examined by multivariate analysis and the odds ratio of each gene is re-scaled to a score between 1 and 5, each gene counts as 1 point when the odds ratio is <1, and so the total score = 40; and
v. Score = 4×TRPV6 + 3×DDX39 + 8×BUB1B + CCR1 + STIL + 3×BLM + 11×C16ORF7 + 4×PIM1 + TPX2 + 2×PTI1 + 2×TCF3 + CCNB1 + DTX2 + 2×ENSA + 5×RCHY1 + 4×NFATC2IP + OBSL1 + 2×MMP15; wherein the genes are examined by multivariate analysis and the odds ratio of each gene is re-scaled to a score between 1 and 11, each gene counts as 1 point when the odds ratio is <1, and so the total score = 56; and
wherein the derived score provides an indication of the likelihood of LRR and/or the likelihood of distant metastasis in the subject.

2. The method according to any claim 1, wherein the first pre-determined reference is a score of 31.

3. The method according to claim 1 or claim 2 wherein the second pre-determined reference is a score of 21.

4. The method according to claim 1, claim 2 or claim 3 wherein the third pre-determined reference is a score of 44.

5. The method according to any of the preceding claims, wherein the step of measuring the expression level of the at least one gene comprises hybridizing the at least one gene with at least one gene probe and measuring the expression level of the at least one gene, preferably, the at least one gene probe comprises at least one gene selected from a group consisting of: TRPV6, DDX39, BUB1B, CCR1, STIL, BLM, C160RF7, TPX2, PTI1, TCF3, CCNB1, DTX2, PIM1, ENSA, RCHY1, NFATC2IP, OBSL1, MMP15, preferably, the at least one gene probe is fixed on a microarray chip.

6. The method according to claim 1, wherein the measurement of gene expression level is performed by a microarray or quantitative reverse transcriptase polymerase chain reaction (quantitative RT-PCR).

7. The method according to any of the preceding claims, wherein the subject has one of the following conditions: zero nodes, one to three positive nodes, and more than three positive nodes.

8. A method according to any of claims 1 to 7, wherein the predicted likelihood of LRR and/or distant metastasis is used to predict or determine the type of adjuvant treatment for the subject following mastectomy and/or breast conserving surgery.

## Patentansprüche

1. Verfahren zum Vorhersagen der Wahrscheinlichkeit von lokoregionärem Rezidiv (LRR) und/oder Fernmetastasen bei einem Individuum mit Brustkrebs nach einer Mastektomie und/oder einer brusterhaltenden Operation, das Folgendes umfasst:
i) das Messen des Expressionsausmaßes von zumindest einem Gen oder einer Vielzahl von Genen in einer aus dem Individuum isolierten Probe, wobei das zumindest eine Gen aus der aus TRPV6, DDX39, BUB1B, CCR1, STIL, BLM, C16ORF7, TPX2, PTI1, TCF3, CCNB1, DTX2, PIM1, ENSA, RCHY1, NFATC2IP, OBSL1 und MMP15 bestehenden Gruppe ausgewählt ist;
ii) das Ableiten eines Werts auf der Grundlage des gemessenen Expressionsausmaßes des zumindest einen Gens oder der Vielzahl von Genen, wobei das Ableiten des Werts unter Verwendung eines prädiktiven Klassifikationsmodells durchgeführt wird, wobei das prädiktive Klassifikationsmodell zumindest einen Scoring-Algorithmus umfasst; und
iii) das Einstufen des Individuums in eine der folgenden Gruppen auf Grundlage des abgeleiteten Werts:
(i) eine Gruppe mit niedrigem LRR-Risiko und/oder Fernmetastasen-Risiko, wenn der abgeleitete Wert kleiner als ein erster vorbestimmter Referenzwert ist; eine Gruppe mit niedrigem LLR-Risiko und/oder Fernmetastasen-Risiko, wenn der abgeleitete Wert gleich oder größer als der erste vorbestimmte Referenzwert ist;
(ii) eine Gruppe mit geringem Fernmetastasen-Risiko und/oder LRR-Risiko, wenn der abgeleitete Wert kleiner als ein zweiter vorbestimmter Referenzwert ist;
(iii) eine Gruppe mit hohem Fernmetastasen-Risiko und/oder LRR-Risiko, wenn der abgeleitete Wert gleich oder größer als ein dritter vorbestimmter Referenzwert ist; und/oder
(iv) eine Gruppe mit mittlerem Fernmetastasen-Risiko und/oder LRR-Risiko, wenn der abgeleitete Wert zwischen dem zweiten vorbestimmten Referenzwert (einschließlich desselben) und dem dritten vorbestimmten Referenzwert liegt;
wobei der zumindest eine Scoring-Algorithmus aus der aus Folgendem bestehenden Gruppe ausgewählt ist:
i) Wert = TRPV6 + DDX39 + BUB1B + CCR1 + STIL + BLM + C16ORF7 + TPX2 + PTI1 + TCF3 + CCNB1 + DTX2 + PIM1 + ENSA + RCHY1 + NFATC2IP + OBSL1 + MMP15;
wobei jedes Gen als ein Punkt zählt, wenn die Hazard Ratio < 1 ist und der Gesamtwert = 18 ist;
ii) Wert = TRPV6 + DDX39 + BUB1B + CCR1 + STIL + BLM + 2×C16ORF7 + TPX2 + PTI1 + TCF3 + CCNB1 + DTX2 + PIM1 + ENSA + RCHY1 + 2×NFATC2IP + OBSL1 + 2xMMP15; wobei, wenn das Hazard Ratio < 1 ist, jedes Gen als ein Punkt zählt, wenn die Gene mittels univariater Analyse untersucht werden, und jedes Gen als zwei Punkte zählt, wenn die Gene mittels multivariater Analyse untersucht werden, und dadurch der Gesamtwert = 21 ist;
iii) Wert = 3×TRPV6 + 5×DDX39 + 18×BUB1B + 4×CCR1 + 4×STIL + 7×BLM + 7×C16ORF7 + 4×PMI1 + 9×TPX2 + 8×PTI1 + 3xTCF3 + 7×CCNB1 + 2×DTX2 + 2xENSA + 5×RCHYI + 6×NFATC2IP + 2×OBSL1 + 6×MMP15; wobei die Gene mittels univariater Analyse untersucht werden und der Wert jedes Gens entsprechend seiner Gewichtung neu skaliert wird und dadurch der Gesamtwert = 102 ist;
iv) Wert = 2×TRPV6 + 2×DDX39 + 2×BUB1B + CCR1 + STIL + 2×BLM + 5×C16ORF7 + 3×PIM1 + 3xTPX2 + 5×PTI1 + TCF3 + CCNB1 + DTX2 + 2xENSA + 3×RCHYI + 4×NFATC2IP + OBSL1 + MMP15; wobei die Gene mittels multivariater Analyse untersucht werden und das Chancenverhältnis jedes Gens auf einen Wert zwischen 1 und 5 neu skaliert wird, wobei jedes Gen als 1 Punkt zählt, wenn das Chancenverhältnis < 1 ist, und dadurch der Gesamtwert = 40 ist; und
v) Wert = 4×TRPV6 + 3×DDX39 + 8×BUB1 B + CCR1 + STIL + 3×BLM + 11×C16ORF7 + 4×PIM1 + TPX2 + 2×PTI1 + 2xTCF3 + CCNB1 + DTX2 + 2xENSA + 5×RCHYI + 4×NFATC2IP + OBSL1 + 2×MMP15; wobei die Gene mittels multivariatet Analyse untersucht werden und das Chancenverhältnis jedes Gens auf einen Wert zwischen 1 und 11 neu skaliert wird, wobei jedes Gen als 1 Punkt zählt, wenn das Chancenverhältnis < 1 ist, und dadurch das Gesamtergebnis = 56 ist; und
wobei der abgeleitete Wert einen Hinweis auf die Wahrscheinlichkeit von LRR und/oder die Wahrscheinlichkeit von Fernmetastasen bei dem Individuum bereitstellt

2. Verfahren nach Anspruch 1, wobei der erste vorbestimmte Referenzwert ein Wert von 31 ist.

3. Verfahren nach Anspruch 1 oder 2, wobei der zweite vorbestimmte Referenzwert ein Wert von 21 ist.

4. Verfahren nach Anspruch 1, 2 oder 3, wobei der dritte vorbestimmte Referenzwert ein Wert von 44 ist.

5. Verfahren nach einem der vorangegangenen Ansprüche, wobei der Schritt des Messens des Expressionsausmaßes des zumindest einen Gens das Hybridisieren des zumindest einen Gens mit zumindest einer Gensonde und das Messen des Expressionsausmaßes des zumindest einen Gens umfasst, wobei die zumindest eine Gensonde vorzugsweise zumindest ein Gen umfasst, das aus der aus TRPV6, DDX39, BUB1B, CCR1, STIL, BLM, C16ORF7, TPX2, PTI1, TCF3, CCNB1, DTX2, PIM1, ENSA, RCHY1, NFATC2IP, OBSL1 und MMP15 bestehenden Gruppe ausgewählt ist, wobei die zumindest eine Gensonde vorzugsweise auf einem Microarray-Chip fixiert ist.

6. Verfahren nach Anspruch 1, wobei das Messen des Genexpressionsausmaßes mittels eines Microarrays oder einer quantitativen Reverse Transkriptase-Polymerase-Kettenreaktion (quantitative RT-PCR) durchgeführt wird.

7. Verfahren nach einem der vorangegangenen Ansprüche, wobei das Individuum eine der folgenden Bedingungen aufweist: keine Knoten; einen oder drei positive Knoten; mehr als drei positive Knoten.

8. Verfahren nach einem der Ansprüche 1 bis 7, wobei die vorhergesagte Wahrscheinlichkeit von LRR und/oder Fernmetastasen verwendet wird, um die Art der adjuvanten Behandlung für das Individuum nach einer Mastektomie und/oder einer brusterhaltenden Operation vorherzusagen oder zu bestimmen.

## Revendications

1. Procédé pour prédire la probabilité de récidive locorégionale (RLR) et/ou de métastases distantes chez un sujet présentant un cancer du sein suite à une mastectomie et/ou à une chirurgie mammaire conservatrice, comprenant :
i. la mesure du taux d'expression d'au moins un gène ou d'une pluralité de gènes dans un échantillon isolé à partir du sujet, où le au moins un gène ou la pluralité de gènes est sélectionné (e) dans un groupe consistant en : TRPV6, DDX39, BUB1B, CCR1, STIL, BLM, C16ORF7, TPX2, PTI1, TCF3, CCNB1, DTX2, PIM1, ENSA, RCHY1, NFATC2IP, OBSL1, MMP15 ;
ii. le fait de dériver un score en se basant sur le taux d'expression mesuré du au moins un gène ou de la pluralité de gènes, où le fait de dériver le score est réalisé en utilisant un modèle de classification prédictif, le modèle de classification prédictif comprenant au moins un algorithme d'attribution de score ; et
iii. la classification du sujet en se basant sur le score dérivé dans l'un des groupes suivants :
(i) un groupe à faible risque de RLR et/ou de métastases distantes lorsque le score dérivé est inférieur à une première référence prédéterminée ; un groupe à risque élevé de RLR et/ou de métastases distantes lorsque le score dérivé est supérieur ou égal à la première référence prédéterminée ;
(ii) un groupe à faible risque de métastases distantes et/ou de RLR lorsque le score dérivé est inférieur à une deuxième référence prédéterminée ;
(iii) un groupe à risque élevé de métastases distantes et/ou de RLR lorsque le score dérivé est supérieur ou égal à une troisième référence prédéterminée ; et/ou
(iv) un groupe à risque intermédiaire de métastases distantes et/ou de RLR lorsque le score dérivé est compris entre la deuxième référence prédéterminée (incluse) et la troisième référence prédéterminée ;
dans lequel le au moins un algorithme d'attribution de score est sélectionné dans un groupe consistant en :
i. Score = TRPV6 + DDX39 + BUB1B + CCR1 + STIL + BLM + C16ORF7 + TPX2 + PTI1 + TCF3 + CCNB1 + DTX2 + PIM1 + ENSA + RCHY1 + NFATC2IP + OBSL1 + MMP15 ; dans lequel chaque gène compte pour un point lorsque le rapport de risque est < 1, et le score total = 18 ;
ii. Score = TRPV6 + DDX39 + BUB1B + CCR1 + STIL + BLM + 2xC16ORF7 + TPX2 + PTI1 + TCF3 + CCNB1 + DTX2 + PIM1 + ENSA + RCHY1 + 2xNFATC2IP + OBSL1 + 2xMMP15 ; dans lequel lorsque le rapport de risque est < 1, chaque gène compte pour un point si les gènes sont examinés par une analyse univariée, et chaque gène compte pour deux points si les gènes sont examinés par une analyse multivariée, et donc le score total = 21 ;
iii. Score = 3xTRPV6 + 5xDDX39 + 18xBUB1B + 4xCCR1 + 4xSTIL + 7xBLM + 7xC16ORF7 + 4xPIM1 + 9xTPX2 + 8xPTI1 + 3xTCF3 + 7xCCNB1 + 2xDTX2 + 2xENSA + 5xRCHY1 + 6xNFATC2IP + 2xOBSL1 + 6xMMP15 ; dans lequel les gènes sont examinés par une analyse univariée et le score de chaque gène est réajusté en fonction de sa pondération, et donc le score total = 102 ;
iv. Score = 2xTRPV6 + 2xDDX39 + 2xBUB1B + CCR1 + STIL + 2xBLM + 5xC16ORF7 + 3xPIM1 + 3xTPX2 + 5xPTI1 + TCF3 + CCNB1 + DTX2 + 2xENSA + 3xRCHY1 + 4xNFATC2IP + OBSL1 + MMP15 ; dans lequel les gènes sont examinés par une analyse multivariée et le rapport des cotes de chaque gène est réajusté à un score compris entre 1 et 5, chaque gène compte pour 1 point lorsque le rapport des cotes est < 1, et donc le score total = 40 ; et
v. Score = 4xTRPV6 + 3xDDX39 + 8xBUB1B + CCR1 + STIL + 3xBLM + 11xC16ORF7 + 4xPIM1 + TPX2 + 2xPTI1 + 2xTCF3 + CCNB1 + DTX2 + 2xENSA + 5xRCHY1 + 4xNFATC2IP + OBSL1 + 2xMMP15 ; dans lequel les gènes sont examinés par une analyse multivariée et le rapport des cotes de chaque gène est réajusté à un score compris entre 1 et 11, chaque gène compte pour 1 point lorsque le rapport des cotes est < 1, et donc le score total = 56 ; et
dans lequel le score dérivé fournit une indication de la probabilité de RLR et/ou la probabilité de métastases distantes chez le sujet.

2. Procédé selon la revendication 1, dans lequel la première référence prédéterminée est un score de 31.

3. Procédé selon la revendication 1 ou la revendication 2, dans lequel la deuxième référence prédéterminée est un score de 21.

4. Procédé selon la revendication 1, la revendication 2 ou la revendication 3, dans lequel la troisième référence prédéterminée est un score de 44.

5. Procédé selon l'une quelconque des revendications précédentes, dans lequel l'étape consistant à mesurer le taux d'expression du au moins un gène comprend l'hybridation du au moins un gène avec au moins une sonde de gène et la mesure du taux d'expression du au moins un gène, de préférence, la au moins une sonde de gène comprend au moins un gène sélectionné dans un groupe consistant en : TRPV6, DDX39, BUB1B, CCR1, STIL, BLM, C16ORF7, TPX2, PTI1, TCF3, CCNB1, DTX2, PIM1, ENSA, RCHY1, NFATC2IP, OBSL1, MMP15, de préférence, la au moins une sonde de gène est attachée sur une puce à microréseau.

6. Procédé selon la revendication 1, dans lequel la mesure du taux d'expression génique est réalisée par un microréseau ou une réaction en chaîne par polymérase-transcriptase inverse quantitative (RT-PCR quantitative).

7. Procédé selon l'une quelconque des revendications précédentes, dans lequel le sujet présente une des conditions suivantes : zéro ganglion, un à trois ganglions positifs, et plus de trois ganglions positifs.

8. Procédé selon l'une quelconque des revendications 1 à 7, dans lequel la probabilité prédite de RLR et/ou de métastases distantes est utilisée pour prédire ou déterminer le type de traitement adjuvant pour le sujet suite à une mastectomie et/ou à une chirurgie mammaire conservatrice.
